# EUROPÄISCHE PATENTSCHRIFT

(11) **EP 3 019 582 B1**
(45) Veröffentlichungstag und Bekanntmachung des Hinweises auf die Patenterteilung: **06.09.2017**
(21) Anmeldenummer: 14747853.1
(22) Anmeldetag: 08.07.2014
(51) Int. Cl.: C10L 3/08, C10L 3/00, C07C 29/00

(54) **FLEXIBEL BETREIBBARES KRAFTWERK UND VERFAHREN ZU DESSEN BETRIEB**
FLEXIBLY OPERABLE POWER PLANT AND METHOD FOR THE OPERATION THEREOF
CENTRALE À FONCTIONNEMENT FLEXIBLE ET SON PROCÉDÉ D'EXPLOITATION

(30) Priorität: 09.07.2013 DE 102013107259; 12.03.2014 DE 102014103311; 09.04.2014 DE 102014105067
(43) Veröffentlichungstag der Anmeldung: 18.05.2016
(73) Patentinhaber: Mitsubishi Hitachi Power Systems Europe GmbH, 47059 Duisburg (DE)
(72) Erfinder: BERGINS, Christian, 45711 Datteln (DE); BUDDENBERG, Torsten, 47447 Moers (DE); KOYTSOUMPA, Efthymia-Ioanna, 45071 Duisburg (DE)
(74) Vertreter: Viering, Jentschura & Partner mbB Patent- und Rechtsanwälte
(86) Internationale Anmeldenummer: PCT/EP2014/064627
(87) Internationale Veröffentlichungsnummer: WO 2015/010895

(56) Entgegenhaltungen:
- WO-A1-2010/069622
- DE-A1-102006 034 712
- FR-A1- 2 939 450
- US-A1- 2011 041 740

## Beschreibung

Die Erfindung richtet sich auf ein Kraftwerk, das einen mit kohlenstoffbefeuerten Brennern und/oder einer Gasturbine ausgestatteten Großdampferzeuger mit angeschlossenem Wasser/Dampfkreislauf aufweist, der und mindestens einen dampfbeaufschlagten Turbosatz mit mindestens einem angeschlossenen Generator umfasst, wobei in dem mit den kohlenstoffbefeuerten Brennern ausgestatteten Großdampferzeuger ein CO₂-haltiger Abgasstrom erzeugt wird, und das mindestens eine Einrichtung zur Erzeugung eines CO₂-reichen Gasstroms umfasst, und das mit seinem den mindestens einen Generator umfassenden stromerzeugenden Teil an ein öffentliches Stromnetz angeschlossen ist, das Regelleistung bereitstellt, wobei die elektrische Leistungsabgabe des stromerzeugenden Teils an das Stromnetz einer stromnetzseitig gesteuerten Leistungsregelung, insbesondere einer Primärregelung und/oder Sekundärregelung und/oder Tertiärregelung und/oder Quartärregelung, unterliegt. Weiterhin richtet sich die Erfindung auf ein Verfahren zum flexiblen Betrieb eines solchen Kraftwerks.

Kraftwerke verlieren heute durch zunehmende und vorrangige Einspeisung erneuerbarer Energien wertvolle Zeiten zur Produktion und Einspeisung von Strom in die Leitungsnetze, da sie bei entsprechend hoher Einspeisung erneuerbarer Energien heruntergefahren werden müssen. Hierdurch leidet die Wirtschaftlichkeit der Kraftwerke, da weniger Strom verkauft werden kann, als produktionstechnisch produzierbar wäre. Gleichzeitig müssen die Kraftwerke betrieben werden, um Netzdienstleistungen zu liefern, ohne dass die eingespeiste Minimalleistung gebraucht wird bzw. adäquat bezahlt wird, da bei vorhandenem Stromüberschuss im Netz die Börsenstrompreise niedriger als die Grenzkosten der Erzeugung sind. Neben der Abregelung erneuerbarer Energien wird deshalb heute bereits das Demand Side Management in Industrieanlagen und die Abregelung von großen Solarkraftwerken und Windparks zur Netzstabilisierung eingesetzt.

Aufgrund des steigenden Anteils erneuerbarer Energien in der Stromversorgung ergeben sich oft Situationen, in denen vorhandene thermische Kraftwerke Ihre Last stark absenken müssen, da die erneuerbaren Energien Einspeisevorrang haben. Hierdurch reduziert sich über das Jahr gesehen der Stromverkauf der thermischen Kraftwerke. Zudem hat durch die teilweise Überproduktion von Strom ein Preisverfall für elektrischen Strom an den Börsen stattgefunden, welcher die Einnahmen derartiger konventioneller Kraftwerke bis hin zur Unrentabilität reduziert.

Die Überproduktion wird zudem dadurch verstärkt, dass thermische Kraftwerke trotzdem, beispielsweise für die Primärregelung, weiterhin laufend am Netz benötigt werden, aber hinsichtlich der Stromproduktion durch die technisch bedingte sogenannte Minimallast in ihrer Leistungs- oder Lastenregelung beschränkt sind. Diese liegt bei großen Braunkohlekraftwerken beispielsweise bei 30-50%, bei Steinkohlekraftwerken bei 15 bis 30% der Nennleistung. So leisten die Kraftwerke zwar Dienste zur Netzstabilisierung, verlieren aber Geld durch die Einspeisung von Strom durch zu niedrige Börsenpreise.

Um hier Abhilfe zu schaffen, sind sogenannte Power to Heat Anwendungen bekannt, bei denen überschüssiger Strom in elektrischen Heißwasser- oder Dampferzeugern eingesetzt wird. Dies kann direkt in der Heizungsanlage von Wohnhäusern oder in großen Wärmespeichern an Kraftwerken für die spätere Fernwärmeversorgung gespeichert werden. Diese Anwendung hat den Vorteil einer sehr niedrigen Investition. Nachteilig ist hierbei, dass aufgrund der Wärmeverluste nur eine kurze Speicherzeit im Bereich von maximal einigen Tagen möglich ist. Zudem wird bei diesem Verfahren aus der hochwertigen Energieform Strom (reine Exergie) Wärme auf einem niedrigen Exergieniveau hergestellt.

Möglich wäre zur Flexibilisierung und Mindestlastabsenkung im Kraftwerk auch, die erzeugte thermische Energie bereits im Dampfkreislauf des Kraftwerkes zu speichern. Dies könnte in der Form von Dampf in sogenannten Ruths-Speichern im Dampfkreislauf des Kraftwerkes erfolgen. Hier sind aber die speicherbaren Energiemengen und die Zeiten einer Speicherung, die insbesondere im Bereich weniger als 60 min liegt, durchaus gering.

Alternativ ist auch die Wärmespeicherung in der Form von Heißwasser in der Vorwärmstrecke des Dampfkreislaufes von Kraftwerken möglich. Aber auch hier sind die speicherbaren Energiemengen gering. Alternativ ist die Wärmespeicherung bei höherer Temperatur in der Form von heißen, flüssigen Salzen (Temperaturänderung) oder als Phasenwechselenergie von Salzen oder anderen Feststoffen möglich. Hier sind allerdings die Systeme unerprobt und schwierig zu implementieren.

Aus der Praxis ist die Herstellung von Synthesegas mit nachfolgender Herstellung von Wasserstoff und/oder Methan und/oder chemischen Folgeprodukten in sogenannten Brennstoffvergasungsanlagen bekannt, die bei geeigneter Ausgestaltung zur Stromerzeugung auch mit Gasturbinenkraftwerken gekoppelt sein können. Diese sogenannten Integrated Gasification Combined Cycle (IGCC) Anlagen sind allerdings durchaus komplex, kostenintensiv und unflexibel. Insbesondere sind sie langsam beim Wechsel der Betriebsweisen zwischen einer Stromproduktion und einer Chemikalienproduktion (z.B. Methanol) und beim Wechsel der eingesetzten Brennstoffe, da neben dem Brennstoffvergaser auch notwendigerweise vorhandene Komponenten wie eine Gasreinigung/Gasaufbereitung oder eine CO₂ -Abscheidung träge Verfahren und Anlagen darstellen. Zudem reduziert sich die Anlagenverfügbarkeit beim dynamischen Betrieb derartiger Anlagen, wenn nicht sogar die geforderte Verfügbarkeit oder die technologischen Eigenarten der Einzelprozesses den hochgradig dynamischen Betrieb gar unmöglich machen. Zudem werden in diesen Prozessen die auf Basis des aus dem Brennstoff stammenden Kohlenstoffs hergestellten chemischen Folgeprodukte auf direktem chemischen Wege erzeugt, was meist zu höheren Kohlenstoff-Umwandlungswirkungsgraden und damit auch energetischen Umwandlungsgraden führt. So können z.B. bis über 50% des Brennstoffkohlenstoffs zum Produkt Methan überführt werden. Gleichzeitig liegen aber die Investitionskosten pro KWel installierter Leistung zwischen 50 und 100% über denen eines normalen thermischen Kraftwerkes. Zudem gibt es weltweit nur ganz wenige IGCC Anlagen. Aus diesen Gründen wird die Brennstoffvergasung bislang weltweit nur in den Fällen angewendet, in denen hochwertige Chemieprodukte wie Treibstoffe oder Dünger aus festen, kohlenstoffhaltigen Brennstoffen, meist Kohlen, in Anlagen hergestellt werden, die quasi im Grundlastbetrieb gefahren werden.

Ferner ist es bekannt, dass CO₂ eines der Treibhausgase ist, die als eine der Ursachen für die Erwärmung des Erdklimas angesehen werden. Daher gibt es zahlreiche umweltpolitische und technologische Bestrebungen, den CO₂ Ausstoß zu verringern. Eines dieser Konzepte befasst sich mit der Speicherung von CO₂ durch die Umwandlung von CO₂ in Methangas und ist beispielsweise in dem Artikel "New technologies for separation, fixation and conversion of carbon dioxide to mitigate global warming" (Hitachi, Vol. 42 (1993), No. 6, Seiten 255- 260) beschrieben. Hierbei wird das während der Verbrennung von fossilen Brennstoffen entstehende CO₂ aus dem Rauchgas abgeschieden und einer Methanisierung zugeführt, bei der künstliches Erdgas (Methan) entsteht. Die Methanisierung ist eine chemische Reaktion, bei der Kohlenstoffmonoxid (CO) oder Kohlenstoffdioxid (CO₂) in Methan (CH₄) umgewandelt wird. Die Reaktion von Kohlenstoffdioxid zu Methan wird auch als Sabatier-Prozess bezeichnet und wurde 1902 von Paul Sabatier und J.B. Sendersens entdeckt. Bei dieser Reaktion reagiert Kohlenstoffmonoxid oder Kohlenstoffdioxid bei Temperaturen von 300 - 700 °C mit Wasserstoff zu Methan und Wasser. Die Reaktion ist exotherm, muss jedoch durch einen Katalysator beschleunigt werden. Weiterhin offenbart DE 10 2006 034 712 A1 ein Kraftwerk gemäß Oberbegriff des Anspruches 1.

Zudem stellt sich im Zusammenhang mit der Erzeugung erneuerbarer Energie mittels Windkraft oder Solarenergie die Problematik, dass häufig mehr Strom ins Netz eingespeist wird, als aktuell abgerufen wird. Dies führt zu einer Menge sogenannten "Überschussstroms", die verbraucht oder gespeichert werden muss, um die Netzstabilität zu gewährleisten. Auch unabhängig von der Einspeisung von aus einer regenerativen Energiequelle erzeugtem Strom in ein Netz, stellt sich die grundsätzliche Problematik, erzeugten Strom gegebenenfalls speichern zu können, um diese Energie zu einem beliebigen Zeitpunkt nutzen zu können.

In diesem Zusammenhang hat sich das sogenannte "Power to Gas"-Konzept als vorteilhaft erwiesen, bei welchem die Energie mittels Methanisierung chemisch umgewandelt und als Methan (CH₄) gespeichert wird. Hierbei wird der für die Bildung des Methans notwendige Wasserstoff insbesondere mittels einer Elektrolyse erzeugt, die den benötigten Strom aus einer erneuerbaren Energiequelle, wie Windkrafträdern oder Solarzellen, erhält. Als CO₂- oder CO-Quelle bieten sich aufbereitete Rauchgasströme von Kraftwerken oder Industrieanlagen an, in welchen kohlenstoffhaltiger Brennstoff oder kohlenstoffhaltige Einsatzstoffe in eine CO₂- oder CO-haltige Gasatmosphäre überführt werden.

Das "Power to Gas"-Konzept stellt eine sinnvolle Methode zur längerfristigen Energiespeicherung und Vermeidung von unmittelbaren CO₂-Abgaben in die Atmosphäre dar, da das bei der Methanisierung entstehenden Produkt Methan (CH₄) als künstlich erzeugtes Erdgas in bestehenden Infrastruktureinrichtungen (Pipelines, Erdgasspeicher) über Monate hinweg langfristig gespeichert werden kann. Die Wasserstoffherstellung kann per Elektrolyse erfolgen. Der Wasserstoff kann aber auch aus anderen, alternativen Quellen stammen. Das CO₂ kann aus einer Abscheidung aus einem CO₂-reichen Strom, z.B. dem Rauchgasstrom eines Kraftwerks, stammen. Die derart gewonnenen Komponenten H₂ und CO₂ werden in einer Methanisierungsanlage oder einem Methanator per Synthese zu H₂O und CH₄ umgewandelt.

Der Erfindung liegt die Aufgabe zugrunde, eine Lösung zu schaffen, die eine flexible Fahrweise oder Betriebsweise eines mit kohlenstoffhaltigem Brennstoff befeuerten Kraftwerks bereitstellt und die insbesondere eine zeitnahe Anpassung der Kraftwerksleistung an netzseitige Leistungsanforderungen ermöglicht.

Diese Aufgabe wird erfindungsgemäß gelöst durch ein Kraftwerk mit den Merkmalen des Anspruches 1 sowie ein Verfahren zum Betrieb eines solchen Kraftwerks gemäß Anspruch 16.

Zweckmäßige Ausgestaltungen und vorteilhafte Weiterbildungen der Erfindung sind Gegenstand der jeweiligen Unteransprüche.

Die vorstehende Aufgabe wird bei einem Kraftwerk der eingangs näher bezeichneten Art somit dadurch gelöst, dass das Kraftwerk mindestens eine Elektrolyseanlage zur Herstellung von Wasserstoff und mindestens eine Syntheseanlage zur Herstellung von Methanol und/oder Methanolfolgeprodukten aus zumindest CO₂-Anteilen des CO₂-reichen Gasstroms und des in der Elektrolyseanlage erzeugten Wasserstoffs umfasst, und dass die mindestens eine Einrichtung zur Erzeugung eines CO₂-reichen Gasstroms und die mindestens eine Elektrolyseanlage zur Herstellung von Wasserstoff (H₂) und die mindestens eine Syntheseanlage zur Herstellung von Methanol und/oder Methanolfolgeprodukten aus zumindest CO₂-Anteilen des CO₂-reichen Gasstroms und des in der Elektrolyseanlage erzeugten Wasserstoffs mittels stromführender und mittels medienführender Leitungen derart leitungsmäßig miteinander verbunden und verschaltet sind, dass der beim Betrieb des Kraftwerks kraftwerksseitig erzeugte Strom ganz oder teilweise wahlweise zum Betrieb einer, mehrerer oder aller dieser aus der mindestens einen Einrichtung zur Erzeugung eines CO₂-reichen Gasstroms, der mindestens einen Elektrolyseanlage zur Herstellung von Wasserstoff (H₂) und der mindestens einen Syntheseanlage zur Herstellung von Methanol und/oder Methanolfolgeprodukten bestehenden Gruppe an Einrichtungen und Anlagen nutzbar ist.

Bei einem Verfahren zum flexiblen Betrieb eines Kraftwerks nach einem der Ansprüche 1-15 wird die vorstehende Aufgabe dadurch gelöst, dass die mindestens eine Einrichtung zur Erzeugung eines CO₂-reichen Gasstroms und die mindestens eine Elektrolyseanlage zur Herstellung von Wasserstoff (H₂) und die mindestens eine Syntheseanlage zur Herstellung von Methanol und/oder Methanolfolgeprodukten aus zumindest CO₂-Anteilen des CO₂-reichen Gasstroms und des in der Elektrolyseanlage erzeugten Wasserstoffs mit stromführenden und medienführenden Leitungen leitungsmäßig miteinander verbunden und verschaltet sind und werden, so dass der beim Betrieb des Kraftwerks kraftwerksseitig erzeugte Strom ganz oder teilweise wahlweise zum Betrieb einer, mehrerer oder aller dieser aus der Einrichtung zur Erzeugung eines CO₂-reichen Gasstroms, der Elektrolyseanlage zur Herstellung von Wasserstoff (H₂) und der Syntheseanlage zur Herstellung von Methanol und/oder Methanolfolgeprodukten bestehenden Gruppe an Einrichtungen und Anlagen genutzt wird.

Die Erfindung geht in einem ersten Aspekt davon aus, ein mit kohlenstoffhaltigem Brennstoff befeuertes Kraftwerk durch die Integration einer Erzeugung eines CO₂-reichen Gasstrom, insbesondere einer CO₂-Abscheidung, einer Wasserstoff erzeugenden Elektrolyse sowie einer chemischen Synthese zur Herstellung von Methanol und/oder Methanolfolgeprodukten, wie z.B. Dimethylether (DME) oder Benzin, zu flexibilisieren.

Unter Flexibilisierung ist hierbei zu verstehen, dass von dem Kraftwerk neben den Produkten Strom und Netzdienstleistungen, wie z.B. die Primärregelung und die Sekundärregelung, weitere Produkte wie z.B. Methanol, DME, Benzin oder weitere Rohstoffe für die chemische oder petrochemische Industrie oder das Transportwesen (regelmäßig) erzeugt werden. Auch das Produkt Demand Side Management (also die Ermöglichung der Netzlastabsenkung durch Reduzierung des Prozessstrombedarfes) kommt hinzu.

Außerdem ist unter Flexibilisierung zu verstehen, dass durch die Kombination solcher Prozesse die Minimaleinspeisung eines Kraftwerkes oder Kraftwerkstandortes weiter abgesenkt und auf negative Werte reduziert werden kann, ohne dass das Kraftwerk abgeschaltet werden muss. Dies ist besonders vorteilhaft, wenn das Kraftwerk trotz ausreichend im Netz vorhandener Stromproduzenten, z.B. erneuerbarer Stromerzeuger, die ansonsten abgeregelt ("Curtailment") werden müssten, weiter zur Netzregelung und Stabilisierung mit dem Netz verbunden bleiben soll.

Die Erfindung umfasst Kraftwerke, deren kohlenstoffbefeuerte Brenner mit kohlenstoffhaltigen biogenen, nachwachsenden Rohstoffen, Steinkohle, Braunkohlen, kohlenstoffhaltigen Abfallstoffen aus der Industrie, kohlenstoffhaltigen gasförmigen Brennstoffen wie Erdgas, Biogas oder Gemischen von kohlenstoffhaltigen Gasen wie Kuppelgasen aus der Chemieindustrie oder der Stahlerzeugung betrieben werden. Anwendbar ist die Erfindung in Dampfkraftwerken bei denen die Brennstoffe in einem Dampferzeuger verbrannt werden oder auch bei Gasturbinenanlagen oder Gasmotoren, in denen flüssige oder gasförmige kohlenstoffhaltige Brennstoffe verbrannt werden oder auch Kombinationen dieser Kraftwerke, z.B. Gasturbinen und Dampfturbinen Kraftwerke, sogenannte GuD Anlagen. Anwendbar ist die Erfindung auch auf die Abgase von Zementöfen, Anlagen der Papierindustrie und sonstige Verbrennungsprozesse, solange in der entsprechenden Anlage/Vorrichtung eine nachgeschaltete Dampferzeugung und Dampfturbine zur Nutzung zumindest eines Teils der Abwärme zur Stromerzeugung enthalten ist.

Wenn nun die Minimallast des Kraftwerkes durch die kraftwerksseitige Eigenverwendung des erzeugten Stromes erfindungsgemäß in einem sogenannten "Power to Fuel" Prozess (PtF) weiter reduziert wird oder sogar noch Überschussstrom aus dem Netz bezogen wird, kann mit Hilfe des Stromes in einer Wasser-Elektrolyse Wasserstoff (H₂) (alternativ auch durch eine Chlor-Alkali-Elektrolyse) hergestellt werden und zudem Kohlendioxid (CO₂) aus den Rauchgasen abgeschieden werden, was weiter die Stromerzeugung reduziert. Aus diesem CO₂ und H₂ wird erfindungsgemäß in einer chemischen Synthese durch einen katalytischen Prozess z.B. Methanol hergestellt, welches nachfolgend weiterverarbeitet werden kann.

Hierdurch ergibt sich die Möglichkeit, im Kraftwerksbetrieb eine höhere Jahresnutzungsdauer des Kraftwerks zu erreichen und durch die Erweiterung der Produktpalette (Herstellung von Methanol oder Methanolfolgeprodukten) auch (wieder) einen rentablen Betrieb zu erreichen. Dies wird möglich ohne dass sogenannte "Kapazitätsmechanismen" benötigt werden, die ein unrentables Kraftwerk für den Stand-by Betrieb subventionieren, d.h. durch Sonderzahlung an den Kraftwerksbetreiber den Betrieb einer ansonsten unrentablen Kraftwerksanlage zur Stützung der Netzstabilität alimentieren.

Die Erfindung geht also von der Vorstellung aus, den überschüssigen produzierten Strom in der Form von Methanol oder Methanolfolgeprodukten außerhalb des Stromnetzes und des Dampfkreislaufes in der chemischen Energie der Stoffe zu speichern und nachfolgend in geeigneter Weise im Kraftwerk oder außerhalb zu anderen Zwecken zu verwenden.

Die Flexibilisierung des Kraftwerkes wird also einerseits durch eine Produktflexibilisierung erreicht. Dies bedeutet, dass das Kraftwerk nicht nur in Bezug auf die Erzeugung des Produktes "Strom" ausgerichtet ist, sondern zudem auch auf die Produktion der Produkte "Methanol und/oder Methanolfolgeprodukte" ausgerichtet ist. Hierbei ist das Kraftwerk zudem derart ausgestaltet, dass flexibel zwischen der jeweils produzierten Menge an Strom und Methanol oder dessen Folgeprodukten variiert werden kann. Dies lässt sich leicht dadurch erreichen, dass entsprechende stromführende und medienführende Verbindungen zwischen den einzelnen Anlagen oder Einrichtungen geschaltet und gegebenenfalls Speicher oder Zwischenspeicher für das in der jeweiligen Anlage oder Einrichtung erzeugte Produkt oder zu verarbeitende Edukt ausgebildet werden. Andererseits betrifft die Flexibilisierung die Betriebsflexibilisierung, d.h. eine Flexibilisierung der möglichen Betriebsweise eines erfindungsgemäßen Kraftwerkes. Dadurch, dass Bestandteil des Kraftwerkes bzw. der gesamten Kraftwerksanlage eine Wasserstoff erzeugende Elektrolyse ist, ist ein sonst nicht üblicher Stromverbraucher vorhanden, der alternativ zur Einspeisung des kraftwerksseitig erzeugten Stromes in das angeschlossene öffentliche Stromnetz mit kraftwerksseitig erzeugtem Strom betrieben werden kann. Die Elektrolyseanlagen zur Herstellung von Wasserstoff haben den Vorteil, dass diese relativ schnell auf eine Stromaufnahme reagieren und damit hinsichtlich ihrer Strom(leistungs)aufnahme und ihrer Produktions- oder Umwandlungsleistung schnell hochgefahren - oder heruntergefahren - werden können. Auch ist es möglich, in der oder den Elektrolyseanlage(n) im angeschlossenen öffentlichen Stromnetz vorhandenen Überschussstrom zu verwenden. Ebenso sind die erfindungsgemäßen Anlagen und Einrichtungen mit weiteren Stromverbrauchern ausgestattet, die schnell Strom aufnehmen können. Damit lässt sich insbesondere eine kurzfristige, vorzugsweise im Minutenbereich liegende Änderung der Strom(leistungs)aufnahme und der Produktions- oder Umwandlungsleistung der Elektrolyseanlage(n) zur Herstellung von Wasserstoff und/oder der Syntheseanlage(n) zur Herstellung von Methanol und/oder Methanolfolgeprodukten und/oder der/den Einrichtung(en) zur Erzeugung eines CO₂-reichen Gasstromes erzielen.

Die Erfindung zeichnet sich daher in Ausgestaltung des Kraftwerks dadurch aus, dass die mindestens eine Elektrolyseanlage zur Herstellung von Wasserstoff (H₂) oder mehrere Elektrolyseanlagen zur Herstellung von Wasserstoff (H₂) kraftwerksseitig bezüglich ihres Strom(leistungs)aufnahmevermögens und ihrer Wasserstofferzeugungskapazität derart ausgelegt und derart regelbar eingerichtet ist/sind, dass ihre Strom(leistungs)aufnahme und Wasserstofferzeugung in Antwort auf eine netzseitige Leistungsregelungsanforderung an das Kraftwerk kurzfristig, vorzugsweise im Minutenbereich, hoch- oder herunterfahrbar ist. In analoger Weise zeichnet sich das erfindungsgemäße Verfahren in Ausgestaltung dadurch aus, dass die Strom(leistungs)aufnahme und die Wasserstofferzeugung der mindestens einen Elektrolyseanlage zur Herstellung von Wasserstoff (H₂) oder der mehreren Elektrolyseanlagen zur Herstellung von Wasserstoff (H₂) kraftwerksseitig in Antwort auf eine netzseitige Leistungsregelungsanforderung an das Kraftwerk kurzfristig, vorzugsweise im Minutenbereich, hoch- oder herunter gefahren wird.

Von Vorteil ist es hierbei, wenn nicht nur die Elektrolyseanlage, sondern auch die Einrichtung(en) zur Erzeugung eines CO₂-reichen Gasstromes und die Syntheseanlage zur Herstellung von Methanol und/oder Methanolfolgeprodukten die flexible Fahr- oder Betriebsweise des Kraftwerks unterstützen. Daher zeichnet sich eine Weiterbildung des Kraftwerks dadurch aus, dass die mindestens eine Einrichtung zur Erzeugung eines CO₂-reichen Gasstroms oder mehrere Einrichtungen zur Erzeugung eines CO₂-reichen Gasstroms und/oder die mindestens eine Syntheseanlage zur Herstellung von Methanol und/oder Methanolfolgeprodukten aus zumindest Teilen des CO₂-reichen Gasstroms oder mehrere Syntheseanlagen zur Herstellung von Methanol und/oder Methanolfolgeprodukten aus zumindest Teilen des CO₂-reichen Gasstroms kraftwerksseitig bezüglich ihres Strom(leistungs)aufnahmevermögens und ihrer Produktions- oder Umwandlungskapazität derart ausgelegt und regelbar eingerichtet ist/sind, dass ihre jeweilige Strom(leistungs)aufnahme und Produktions- oder Umwandlungsleistung in Antwort auf eine netzseitige Leistungsregelungsanforderung an das Kraftwerk kurzfristig, vorzugsweise im Minutenbereich, hoch- oder herunterfahrbar ist.

Unter einem kurzfristigen, vorzugsweise im Minutenbereich liegenden Hoch- oder Herunterfahren einzelner oder mehrerer Anlagen oder Einrichtungen des Kraftwerks wird vorstehend und nachfolgend im Rahmen dieser Anmeldung verstanden, dass als Reaktion auf eine netzseitige Primärregelungsanforderung ein Hoch- oder Herunterfahren innerhalb von 30 s und als Reaktion auf eine netzseitige Sekundärregelungsanforderung ein Hoch- oder Herunterfahren innerhalb von 5 min erfolgt, sofern im Einzelfall in der nachfolgenden Beschreibung keine anderslautenden Angaben gemacht werden.

In gleicher Weise ist in Weiterbildung des erfindungsgemäßen Verfahrens vorgesehen, dass die jeweilige Strom(leistungs)aufnahme und Produktions- oder Umwandlungsleistung der mindestens einen Einrichtung zur Erzeugung eines CO₂-reichen Gasstroms oder mehrerer Einrichtungen zur Erzeugung eines CO₂-reichen Gasstroms und/oder der mindestens einen Syntheseanlage zur Herstellung von Methanol und/oder Methanolfolgeprodukten aus zumindest Teilen des CO₂-reichen Gasstroms oder mehrerer Syntheseanlagen zur Herstellung von Methanol und/oder Methanolfolgeprodukten aus zumindest Teilen des CO₂-reichen Gasstroms kraftwerksseitig in Antwort auf eine netzseitige Leistungsregelungsanforderung an das Kraftwerk kurzfristig, vorzugsweise im Minutenbereich, hoch- oder heruntergefahren wird.

Da sich bei dem erfindungsgemäßen Kraftwerk der mittels des mindestens einen Generators erzeugte Strom sehr schnell und kurzfristig nicht nur ins angeschlossene Stromnetz einspeisen, sondern auch auf die erfindungsgemäß vorhandenen Anlagen und Einrichtungen verteilen lässt, kann ein erfindungsgemäßes Kraftwerk eine schnelle Laständerung vornehmen. Die Erfindung zeichnet sich daher weiterhin dadurch aus, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms kraftwerksseitig hinsichtlich ihres jeweiligen Strom(leistungs)aufnahmevermögens und ihrer jeweiligen Produktions- oder Umwandlungsleistung derart ausgelegt und regelungstechnisch miteinander verbunden sind, dass sie in Antwort auf eine netzseitige Leistungsregelungsanforderung an das Kraftwerk im Verbund jeweils bezüglich ihrer jeweiligen Strom(leistungs)aufnahme und Produktions- oder Umwandlungsleistung derart kurzfristig, vorzugsweise im Minutenbereich, hoch- oder herunterfahrbar sind, dass das Kraftwerk im Falle einer netzseitigen Leistungsregelungsanforderung leistungsmäßig im Wege einer Laständerung mit einem Laständerungsgradient im Bereich von 3%/min - 30%/min an die geänderte Leistungsanforderung anpassbar ist.

Um eine besonders schnelle und kurzfristige Anpassung der Strom(leistungs)aufnahme der einzelnen Anlagen/Anlagenteile oder Einrichtungen zu ermöglichen, ist es zweckmäßig, wenn diese mit für eine dauerhafte Nennbelastung oder einen normalen Auslegungswert oder normalen Betriebswert ausgelegt sind, aber kurzfristig mit einer demgegenüber deutlich höheren Spitzenbelastung betrieben werden können. In Ausgestaltung sieht die Erfindung für das Kraftwerk daher weiterhin vor, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms bezüglich ihrer jeweiligen Strom(leistungs)aufnahme und/oder ihrer jeweiligen Produktions- oder Umwandlungsleistung derart ausgelegt ist/sind, dass sie, insbesondere in Antwort auf eine netzseitige Leistungsregelungsanforderung an das Kraftwerk, kurzzeitig im Minutenbereich, vorzugsweise über einen Zeitraum von bis zu 30 Minuten, mit einer Strom(leistungs)aufnahme beaufschlagbar ist/sind, die 100 - 300 %, vorzugsweise 150 - 200 %, des Normalauslegungs- oder Normalbetriebswertes der jeweiligen Anlage oder Einrichtung beträgt.

Um eine besonders gute Flexibilisierung des Kraftwerkes zu erreichen, ist es hilfreich, wenn die einzelnen Anlagen oder Einrichtungen einzeln und individuell sowohl in Bezug auf ihre Stromaufnahme oder Strom(leistungs)aufnahme als auch in Bezug auf ihre Produktleistung oder Umwandlungsleistung an unterschiedliche Betriebsfahrweisen des Kraftwerkes anpassbar sind oder solche erlauben. Die Erfindung zeichnet sich daher in weiterer Ausgestaltung des Kraftwerkes auch dadurch aus, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms bezüglich ihrer jeweiligen Strom(leistungs)aufnahme und ihrer jeweiligen Produktions- oder Umwandlungsleistung individuell ansteuerbar und regelbar ausgebildet sind. In analoger Weise ist in Ausgestaltung des erfindungsgemäßen Verfahrens vorgesehen, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms bezüglich ihrer jeweiligen Strom(leistungs)aufnahme und ihrer jeweiligen Produktions- oder Umwandlungsleistung individuell angesteuert und geregelt werden.

Um ein Kraftwerk, bei dem ein CO₂-haltiges Abgas erzeugt wird, im Sinne der vorliegenden Erfindung flexibel betreiben zu können, ist es vorteilhaft und zweckmäßig, wenn die mindestens eine Syntheseanlage zur Herstellung von Methanol und/oder Methanolfolgeprodukten oder die mehreren Syntheseanlagen zur Herstellung von Methanol und/oder Methanolfolgeprodukten insgesamt kapazitätsmäßig derart ausgelegt ist/sind, dass damit 10 - 50 Gew.-%, insbesondere 30 - 40 Gew.-%, bevorzugt 35 Gew.-%, des bei Volllast des Kraftwerks entstehenden und in dem CO₂-haltigen Abgasstrom enthaltenen CO₂ zu Methanol und/oder einem Methanolfolgeprodukt umwandelbar ist, was die Erfindung ebenfalls vorsieht.

Zweckmäßig und vorteilhaft ist es zudem auch, dass die mindestens eine Syntheseanlage zur Herstellung von Methanol und/oder Methanolfolgeprodukten oder die mehreren Syntheseanlagen zur Herstellung von Methanol und/oder Methanolfolgeprodukten insgesamt bezüglich ihres Strom(leistungs)aufnahmevermögens und der jeweils möglichen Produktions- oder Umwandlungsleistung kapazitätsmäßig derart ausgelegt ist/sind, dass maximal der bei Volllast und/oder Maximalleistung des Kraftwerks von diesem insgesamt erzeugbare elektrische Strom für die Herstellung von Methanol und/oder Methanolfolgeprodukte nutzbar ist, wodurch sich das Kraftwerk in Ausgestaltung ebenfalls auszeichnet.

Bei der Einrichtung zur Erzeugung eines CO₂-Gasstromes kann es sich insbesondere um CO₂-Abscheideanlagen handeln, die das CO₂ (Kohlendioxid) aus dem bei der Verbrennung von kohlenstoffhaltigem Brennstoff entstehenden Abgas herauswaschen oder herausfiltern oder gewinnen. Das Kraftwerk zeichnet sich daher in weiteren Ausgestaltung auch dadurch aus, dass die mindestens eine Einrichtung zur Erzeugung eines CO₂-reichen Gasstroms oder die mehreren Einrichtungen zur Erzeugung eines CO₂-reichen Gasstroms mindestens eine CO₂-Abscheideanlage, insbesondere eine Post Combustion Capture (PCC)-Anlage und/oder einen oder mehrere nach dem Oxyfuel-Prozess betriebene(n) Brenner oder Brennereinrichtungen des Großdampferzeugers mit zugeordneter CO₂-Abscheideanlage, umfasst/umfassen oder aus diesen besteht/bestehen. Bei einem Oxyfuel-Betrieb der Brenner kann der dafür benötigte Sauerstoff vorteilhafter Weise insbesondere auch aus einer (der) Elektrolyseanlage(n) zur Herstellung/Erzeugung von Wasserstoff stammen, in welcher/welchen Wasser unter der Erzeugung von Sauerstoff (O₂) zu Wasserstoff (H₂) umgesetzt wird.

Von Vorteil ist es weiterhin, wenn die die Produktflexibilisierung ermöglichenden und betreffenden Anlagen bezüglich ihrer Strom(leistungs)aufnahme und ihrer Produktions- oder Umwandlungsleistung insgesamt derart ausgelegt sind, dass das Kraftwerk mit seiner anlagentechnisch notwendigen Minimallast ohne Stromeinspeisung in das Stromnetz betreibbar ist, also sämtlicher dann entstehender Strom in die entsprechenden, der Produktflexibilisierung dienenden Anlagen und/oder Einrichtungen fließt. Die Erfindung sieht daher weiterhin ein Kraftwerk vor, bei welchem die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms bezüglich ihrer Strom(leistungs)aufnahme und Produktions-oder Umwandlungsleistung insgesamt derart ausgelegt sind, dass bei ihrem Betrieb das Kraftwerk bei einem Betrieb mit seiner anlagentechnisch notwendigen Minimallast ohne Stromeinspeisung in das Stromnetz betreibbar ist.

Es soll aber auch vorgesehen sein, dass das Kraftwerk mit Überschussstrom aus dem angeschlossenen Stromnetz betrieben werden kann. Das erfindungsgemäße Kraftwerk zeichnet sich daher in Ausgestaltung auch dadurch aus, dass das Kraftwerk als Stromsenke für das angeschlossenen öffentliche Stromnetzausgebildet ist, wobei die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms bezüglich ihrer Strom(leistungs)aufnahme und Produktions- oder Umwandlungsleistung insgesamt derart ausgelegt und mit dem Stromnetz verschaltet sind, dass sie mit aus dem Stromnetz bezogenem Überschussstrom betreibbar sind.

Um einen Beitrag zur Leistungsregelung des öffentlichen Stromnetzes leisten zu können, ist in Ausgestaltung der Erfindung weiterhin vorgesehen, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms als abschaltbare Last mit dem öffentlichen Stromnetz verbunden und verschaltet sind.

In Ausgestaltung des erfindungsgemäßen Verfahrens ist in analoger Weise vorgesehen, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten aus zumindest Teilen des CO₂-reichen Gasstroms als mit dem öffentlichen Stromnetz verbundene und verschaltete abschaltbare Last betrieben werden.

Bei der vorgeschlagenen erfindungsgemäßen Flexibilisierung des Kraftwerkes kann es weiterhin vorteilhaft und zweckmäßig sein, auch entstehende Wärme innerhalb des Kraftwerkes in wärmeeinkoppelnder und/oder wärmeauskoppelnder Weise miteinander zu verknüpfen. Eine erfindungsgemäße Möglichkeit besteht gemäß Weiterbildung der Erfindung darin, dass die mindestens eine Elektrolyseanlage zur Herstellung von Wasserstoff (H₂) oder die mehreren Elektrolyseanlagen zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage zur Herstellung von Methanol und/oder Methanolfolgeprodukten aus zumindest Teilen des CO₂-reichen Gasstroms oder die mehreren Syntheseanlagen zur Herstellung von Methanol und/oder Methanolfolgeprodukten in einer beim Betrieb dieser Anlage(n) und/oder Einrichtung(en) im Bereich von 30 - 400°C, vorzugsweise im Bereich von 30 - 150°C, entstehende Abwärme über mindestens eine Abwärme führende Leitung mit einer Vorwärmung des Speisewassers des Wasser/Dampfkreislaufs und/oder einer Vorwärmung einer CO₂-Abscheideanlage, insbesondere einer Post Combustion Capture (PCC) - Anlage, und/oder einer Vorwärmung mindestens eines der in dem Kraftwerk eingesetzten in Edukte und/oder erzeugten Produkte in Leitungsverbindung steht.

Anlagentechnisch ist es weiterhin von Vorteil, wenn der mit der Elektrolyseanlage erzeugbare Wasserstoff ausreicht, das gesamte beim Kraftwerksbetrieb erzeugte oder abgeschiedene Kohlendioxid (CO₂) zu Methanol und/oder einem oder mehreren Methanolfolgeprodukten umzusetzen/umzuwandeln. Die Erfindung sieht daher in weiterer Ausgestaltung des Kraftwerkes vor, dass die mindestens eine Elektrolyseanlage zur Herstellung von Wasserstoff (H₂) oder die mehreren Elektrolyseanlagen zur Herstellung von Wasserstoff (H₂) bezüglich ihrer Produktions- und/oder Umwandlungskapazität derart ausgelegt ist/sind, dass mit der erzeugbaren Wasserstoffmenge der gesamte CO₂-Anteil des bei der Verbrennung kohlenstoffhaltigen Brennstoffs in den Brennern des Großdampferzeugers entstehenden Abgasstroms und/oder das gesamte in der mindestens einen CO₂-Abscheideanlage abgeschiedene CO₂ in der oder den Syntheseanlagen zur Herstellung von Methanol und/oder Methanolfolgeprodukten zu Methanol oder einem Methanolfolgeprodukt umsetzbar ist.

Schließlich zeichnet sich die Erfindung noch dadurch aus, dass jeder der Einrichtungen oder Anlagen aus der Gruppe der mindestens einen Einrichtung zur Erzeugung eines CO₂-reichen Gasstroms, der mindestens einen Elektrolyseanlage zur Herstellung von Wasserstoff (H₂) und der mindestens Syntheseanlage zur Herstellung von Methanol und/oder Methanolfolgeprodukten mindestens ein Edukt- und/oder Produktspeicher, insbesondere der Elektrolyseanlage ein Wasserstoffspeicher und/oder ein Sauerstoffspeicher und der Einrichtung zur Erzeugung eines CO₂-reichen Gasstroms ein CO₂-Speicher, zugeordnet ist. Diese Speicher sind vorzugsweise als Pufferspeicher ausgebildet, so dass die in den einzelnen Anlagen/Einrichtungen ablaufenden Prozesse unabhängig voneinander gefahren werden können und sich die Dynamik der einzelnen Prozesse nicht im Wege steht. Insofern tragen auch die Speicher zur Flexibilisierung des Betriebes oder der Fahrweise des Kraftwerkes bei. Priorität bei der Gewichtung der einzelnen Anlagen/Einrichtungen und der darin jeweils ablaufenden Prozesse oder Verfahren besitzt dabei die Herstellung von Methanol und/oder Methanolfolgeprodukten. Darauf werden die Kapazitäten und Leistungen insbesondere der CO₂-Abscheideanlage(n) und der Elektrolyseanlage(n) jeweils abgestimmt.

Hinsichtlich der Flexibilität des Anlagenbetriebes bietet die hier und im folgenden dargestellte Erfindung der Stromerzeugung in einem mit kohlenstoffhaltigen Brennstoffen befeuerten Kraftwerk mit nachgeschalteter CO₂ Abscheidung (Post Combustion Capture PCC) oder integrierter CO₂ Abscheidung (Oxyfuel) den Vorteil, dass der Kraftwerksprozess höchst flexibel hinsichtlich der Stromerzeugungsmenge gefahren werden kann. Das Verfahren ist kombinierbar mit neu zu bauenden thermischen Kraftwerken oder auch als Erweiterung nachrüstbar an bestehenden Kraftwerksanlagen. Zwar ist der Umwandlungsweg über die Stromerzeugung und Elektrolyse exergetisch nicht optimal, allerdings lassen sich die energetischen und exergetischen Nachteile dieser Verfahrenskombination zumindest zum Teil wieder durch besonders vorteilhafte, energetische Verschaltungen der Prozesse ausgleichen. Mittel der Produktion kohlenstoffhaltiger Energieträger (Methanol oder Methanolfolgeprodukte) lässt sich bei mit kohlenstoffhaltigen Brennstoffen befeuerten Kraftwerken deren Minimallast sogar auf negative Werte (0 bis > -10,0 %) einstellen. Ebenso ist eine Steigerung der Primärregelfähigkeit auf bis über 100 MWₑₗ pro Minute auch bei kleineren Kraftwerkseinheiten möglich.

So bieten sich verschieden Vorteile durch die Wärmeverschaltung nachgeschalteter Verfahrensteile wie der CO₂-Abscheidung nach dem Post Combustion Capture Prinzip, der Wasserstoffelektrolyse und der nachgeschalteten Methanol- oder Methanolfolgeprodukt-Herstellung.

So kann die Abwärme von Anlagenteilen wie der CO₂ Abscheidung oder der Reaktoren (Methanolherstellung oder -umwandlung) energetisch günstig in die Hochdruckvorwärmung oder Niederdruckvorwärmenung des Kraftwerkes eingebunden werden oder auch zur Vorheizung von Reaktionsedukten vor den Reaktoren verwendet werden.

Notwendige Wärme zum Betrieb von Anlagenteilen wie der Desorption des PCC Prozesses oder der evtl. optional nachgeschalteten Rektifikation oder Destillation der Produkte kann aus der Reaktionsabwärme der Reaktoren (Methanolherstellung oder -umwandlung) entnommen werden oder als Anzapfdampf aus dem Dampfprozess energetisch effizient entnommen werden oder zumindest auch teilweise aus der Kühlung von Produkten und Zwischenprodukten gewonnen werden. Hierdurch wird der Umwandlungswirkungsgrad von Strom zu den jeweiligen chemischen Produkten wesentlich auf bis über 70% gesteigert im Vergleich zu weniger als 60% bei Anlagen, die keine derartige Energieintegration besitzen.

Findet die CO₂-Abscheidung durch das PCC Verfahren statt, sollte der chemischen Absorption sinnvollerweise wenn notwendig eine Rauchgasentschwefelung und/oder Rauchgaskühlung vorgeschaltet sein um auch die CO₂-Abscheidung höchst effizient und bei niedrigstem Waschmittelverbrauch (zumeist Aminlösungen) durchzuführen.

Anfallendes Wasser aus der Kühlung von Produkten oder Zwischenprodukten sollte nach einer evtl. notwendigen Aufreinigung vorzugsweise wieder der Wasserstoffelektrolyse zugeführt werden. Die Aufreinigung kann vorzugsweise in den Anlagen zur Speisewasseraufbereitung des Kraftwerkes und/oder auch in einer speziell dafür ausgelegten Wasseraufbereitung erfolgen.

Damit die volle Laständerungsgeschwindigkeit der Elektrolyse zur Unterstützung der Regelfähigkeit des Kraftwerkes oder im Rahmen eines Demand Side Managements verwendet werden kann, ist es zweckmäßig, in den Gesamtprozess Speicher für Wasser und/oder Wasserstoff und/oder CO₂ und/oder Sauerstoff zu integrieren, die eine im Bereich von Sekunden bis zu Stunden verzögerte Laständerung der chemischen Reaktoren oder der CO₂-Abscheidung zulassen. Dies können z.B. Druckspeicher (Druckbehälter oder Kavernen) sein oder auch Flüssigkeitsspeicher. So kann damit die Last der Elektrolyse durch die aufgebrachte elektrische Leistung im Bereich weniger Sekunden um bis zu 100% geändert werden, während die CO₂ Abscheidung und die nachgeschalteten Reaktoren längere Zeit für die Laständerung aufwenden können.

Die Gesamtanlagenteilkomponenten (Anlagenkomplexe Kraftwerk + Wasserstoffelektrolyse + CO₂-Abscheidung + Reaktoren) können entweder antiproportional zum Strombedarf im Netz gefahren werden, d.h. hohe Last der Elektrolyse, CO₂-Abscheidung und/oder der Reaktoren (Methanolherstellung und Umwandlung zu Methanolfolgeprodukten) insbesondere dann, wenn niedriger Strombedarf im Netz herrscht, während das Kraftwerk selbst bei niedrigstmöglicher Last betrieben wird, oder auch entkoppelt in der Art geregelt werden, dass die Wasserstoffelektrolyse, die CO₂-Abscheidung und die Reaktoren im Regelfall bei Maximallast gefahren werden und nur bei positiver Lastanforderung des Stromnetzes abgeregelt werden, d.h. bezüglich ihrer Strom(leistungs)aufnahme heruntergefahren werden (Demand Side Management DSM).

Diese letztgenannte Art des Anlagenbetriebs oder Kraftwerksbetriebs ist insbesondere dann sinnvoll, wenn das Preisniveau an der Strombörse sehr niedrig ist und/oder häufig positive Lastgradienten (Einspeisung) zu fahren sind, da durch ein momentanes und unverzügliches Abschalten der Wasserstoffelektrolyse sehr schnell eine hohe zusätzliche Stromeinspeisung geleistet werden kann.

Im erstgenannten Fall können je nach momentaner Anlagenleistung durch schnellste Laststeigerung oder Lastabsenkung der Wasserstoffelektrolyse Lastgradienten der Kraftwerksleistung in beide Richtungen (positiv oder negativ) unterstützt werden.

Die gesamte Dynamik des Systems ist zudem unterstützbar durch die parallele Schaltung von Batteriesystemen, die sich zweckmäßig auf niedriger Spannungsebene parallel zu Wasserstoffelektrolysen integrieren lassen. Die Größe und Auslegung eines solchen Batteriespeichers kann anhand des erwarteten Strompreisniveaus und der Auslastung des Kraftwerkes sowie der erwarteten Regeleingriffe zur Stabilisierung des Stromnetzes erfolgen.

Auch die zuvor genannten Verfahren zur Speicherung von Wärme im Dampfkreislauf oder aus elektrischer Wärmeerzeugung lassen sich vorteilhaft mit dem erfindungsgemäßen Verfahren kombinieren.

Befindet sich das Kraftwerk nahe einer Industrieanlage, in der Sauerstoff benötig wird, wie z.B. in der Stahlindustrie oder der chemischen Industrie, ist es zweckmäßig, den bei der Durchführung einer Elektrolyse von Wasser entstehenden Sauerstoff in diesen Industrieanlagen zu verwenden und gleichzeitig die Leistung etwaiger dort vorhandener Luftzerlegungsanlagen zu reduzieren.

Ansonsten kann der Sauerstoff teilweise oder vollständig auch zur Unterstützung der Feuerung des Kraftwerkes zur Steigerung des Kesselwirkungsgrades durch die Reduktion des Abgasvolumenstromes oder in einer reinen Oxyfuelfeuerung mit integrierter CO₂-Abscheidung als Alternative zur PCC CO₂-Abscheidung verwendet werden.

Ist weder die Verwendung in einem nahegelegenen Industriebetrieb noch die Verwendung in einer sauerstoffangereicherten Feuerung möglich oder gewünscht lässt sich der Sauerstoff nach evtl. notwendiger Aufreinigung und Trocknung auch höher Verdichten und als Drucksauerstoff oder nach Verflüssigung als flüssiger Sauerstoff vermarkten. Die Implementierung solcher nachgeschalteter Prozesse erhöht weiter die Stromaufnahme des Gesamtprozesses und kann sinnvoll zur Erweiterung des Lastregelbereiches bis hin zu negativen Stromeinspeisungen (= Stromaufnahme) des Kraftwerks bzw. Industriestandortes genutzt werden.

Bei dem hier beschrieben Verfahren zur Flexibilisierung eines Kraftwerkes können je nach Ausgestaltung und verwendetem Brennstoff bei Nutzung der vollen Eigenstromerzeugung unabhängig vom Lastbereich im stationären Betrieb ca. 10-35% des im Brennstoff bzw. Rauchgas enthaltenen Kohlenstoffes in Methanol und Methanolfolgeprodukte umgewandelt werden. Durch die Verwendung von zusätzlich aus dem Netz bezogenem Strom lässt sich dieser Anteil weiter auf bis über 90% erhöhen. Außerdem kann bei Einsatz von Speichern für die Zwischenprodukte CO₂, H₂, O₂ zeitweise der Betrieb von der Stromerzeugung im Kraftwerk entkoppelt werden. Weiterhin ist es auch möglich durch die Auslegung der Wasserstoffelektrolyse auf niedrigere Stromdichten im Normalbetrieb zeitweise kurzfristig die Leistung der Elektrolyse stark bis auf Werte über 200% des Normalbetriebes zu erhöhen, um mehr Strom zu verbrauchen und die Laständerungen der Stromeinspeisung mit diesem negativen Gradienten zu unterstützen.

Die Erfindung ist nachstehend anhand einer Zeichnung beispielhaft näher erläutert. Diese zeigt in
- Fig. 1: in schematischer Darstellung ein Anlagenschaltbild eines erfindungsgemäßen Kraftwerks und in
- Fig. 2: in ebenfalls schematischer Darstellung die Verschaltung eines erfindungsgemäßen Kraftwerkes mit zugeordneten Komponenten.

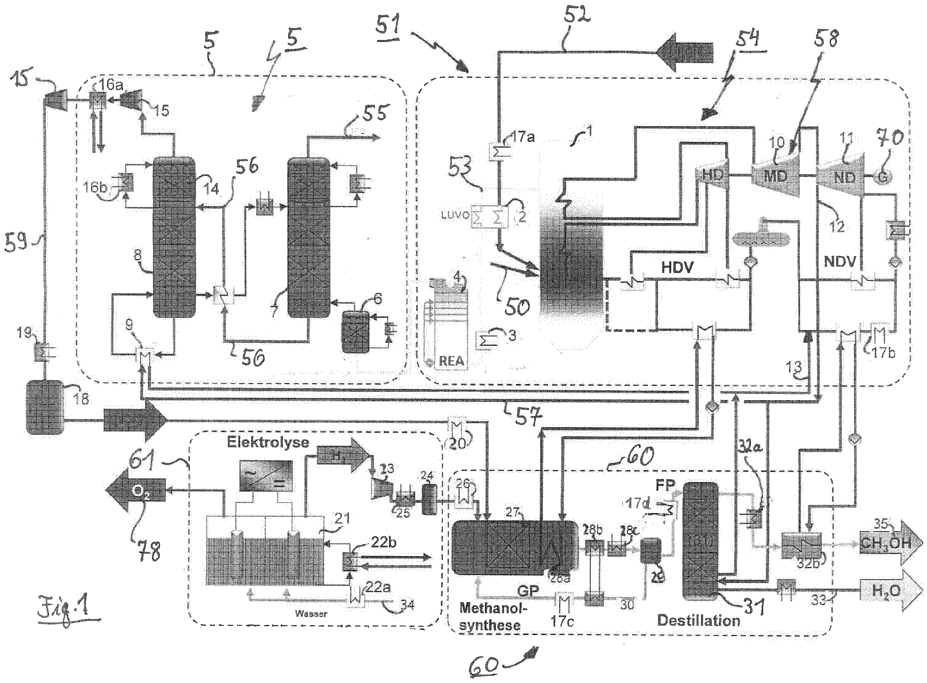

Die Fig. 1 zeigt in schematischer Darstellung ein mit Braunkohle 50 befeuertes Kraftwerk 51, das einen Großdampferzeuger 1 mit angeschlossenem Wasser/Dampf-Kreislauf 54 umfasst. Das bei der Verfeuerung der Braunkohle 50 in den Brennern des Großdampferzeugers 1 entstehende Rauchgas 53 wird in einer Leitung einem Luftvorwärmer 2 zugeführt, den im Gegenstrom die durch eine Leitung zugeführte Verbrennungsluft 52 zugeführt wird, die in dem Luftvorwärmer 2 vorgeheizt wird. Danach wird das Rauchgas 53 einem Wärmeverschubsystem 3 zugeführt und aus dem Rauchgas 53 Wärme ausgekoppelt, die der Speisewasservorwärmung des Wasser/Dampf-Kreislaufes 54 zur Verfügung gestellt wird. Danach wird das Rauchgas 53 leitungsmäßig in eine Rauchgasentschwefelungsanlage 4 geleitet, wo es weitgehend von SO₂ (Schwefeldioxid) und SO₃ (Schwefeltrioxid) befreit wird. Das dermaßen gereinigte Rauchgas 53 verlässt die Rauchgasentschwefelungsanlage 4 mit einer Temperatur von 40-90 °C. Um eine hohe Verfügbarkeit und hohe Abscheideraten in der dem Dampferzeuger 1 nachgeschalteten Post Combustion Capture(PCC)-CO₂-Abscheideanlage 5 zu erzielen und zu gewährleisten, wird das Rauchgas 53 zunächst noch einer Feinreinigung in einer Feinreinigungsanlage 6 unterzogen. Die Feinreinigungsanlage 6 ist als Rauchgaskühler mit zugeordnetem NaOH (Natriumhydroxid)-Vorwäscher ausgebildet, in der eine Wäsche des Rauchgases 53 mit einer NaOH-Lösung stattfindet und das Rauchgas 53 auf eine Temperatur von 30-50 °C abgekühlt wird. Gleichzeitig wird die SO₂/SO₃-Konzentration des Rauchgases 53 weiter erniedrigt.

Von der Feinreinigungsanlage 6 aus wird das gekühlte Rauchgas 53 in einen Absorber 7 der Post Combustion Capture (PCC)-Anlage 5 eingeleitet und darin im Gegenstrom mit einem das CO₂ aus dem Gasstrom herauslösenden Waschmittel in Kontakt gebracht. Bei dem CO₂-Waschmittel handelt es sich im Ausführungsbeispiel um eine wässrige Aminlösung, die als einfache Monoethanolaminlösung ausgebildet ist, so dass der Energiebedarf bei der nachfolgenden Desorption im Desorber 8 3,2-3,8 MJ/kg entfernten Kohlendioxids beträgt. Es ist aber auch möglich, ein CO₂-Waschmittel einzusetzen, das in Bezug auf den bei der Desorption notwendigen Energiebedarf derart optimiert ist, dass dort lediglich nur noch ein Energiebedarf im Bereich von 2,4-2,8 MJ/kg entfernten Kohlendioxids benötigt wird. Den Absorber verlässt einerseits ein gereinigtes Gas 55 und andererseits die mit CO₂ gesättigte CO₂-Waschmittellösung, die über eine Leitung 56 dem ebenfalls einen Bestandteil der Post Combustion Capture (PCC)-Anlage 5 ausbildenden Desorber 8 zugeführt wird. Die für die Desorption im Desorber 8 benötigte Wärme wird auf übliche Art und Weise in einem Reboiler 9 in Form von Dampf bereitgestellt und zugeführt. Dieser Dampf wird im Ausführungsbeispiel als Anzapfdampf 12 zwischen einer Mitteldruckturbine 10 und einer Niederdruckturbine 11 des im Wasser/Dampf-Kreislauf 54 angeordneten Turbosatzes 58 bei einer Temperatur von 110 °C und 200 °C dem Wasser/DampfKreislauf 54 entnommen und über eine Leitung 57 dem Reboiler 9 zugeführt. Das im Reboiler 9 bei der Reboilerbeheizung entstehende Kondensat wird über eine Leitung 13 in die Vorwärmstrecke des Wasser/Dampf-Kreislaufes 54 zurückgeführt. Den Desorber 8 verlässt einerseits das vom CO₂ befreite Waschmittel, das in üblicher Weise im Kreislauf zum Absorber 7 zurückgeführt wird, und andererseits ein Gemisch aus Kohlendioxid (CO₂) und Wasserdampf. Dieses Kohlendioxid/Wasserdampf-Gemisch wird nach einer im Ausgangsbereich des Desorbers 8 angeordneten Kühlung und Nachwäsche 14 einer Verdichterstufe 15 zugeleitet. Die Kühlung im Kopfbereich des Desorbers 8 erfolgt mit Hilfe eines Wärmetauschers 16b und die Nachwäsche 14 erfolgt vorzugsweise mittels eines sauren Mediums. In der Verdichterstufe 15 wird das Kohlendioxid/Wasserdampfgemisch auf einen Druck oberhalb von 20 bar, vorzugsweise auf einen Druck zwischen 30-60 bar, verdichtet. Die fühlbare Wärme des den Desorber 8 und die Verdichterstufe 15 verlassenden Kohlendioxid/Wasserdampfgemisches und teilweise auch die Kondensationswärme des darin enthaltenen Wassers wird in einem der Verdichterstufe nachgeschalteten und von dem Kohlendioxid/Wasserdampfgemisch durchströmten Wärmetauscher 16a sowie dem dem Ausgangsbereich des Desorbers 8 zugeordneten Wärmetauscher 16b entnommen oder entkoppelt. Die hier entnommene oder entkoppelte Wärmeenergie wird beispielsweise über Wärmetauscher 17a, 17b, 17c, 17d der Niederdruckvorwärmung (17b) des Wasser/Dampf-Kreislaufes 54, der Verbrennungsluftvorwärmung (17a) oder Eduktvorwärmungen (17c, 17d), die im Bereich der Reaktoren (27, 31) für die Methanolsynthese und die Destillation einer Syntheseanlage 60 zur Herstellung von Methanol und/oder Methanolfolgeprodukten zugeführt. Bei der im Ausführungsbeispiel mehrere Verdichterstufen 15 umfassenden Verdichteranlage ist der Wärmetauscher 16a zwischen der ersten und der letzten Verdichterstufe 15 angeordnet. Der die letzte Verdichterstufe 15 verlassende CO₂-reiche Gasstrom 59 wird einem Speicher 18 und von daraus der Syntheseanlage 60 zur Herstellung von Methanol und/oder Methanolfolgeprodukten zugeführt. Vor dem Eintritt in den Speicher 18 durchläuft der CO₂-reiche Gasstrom nochmals einen Wärmetauscher 19, in welchem dieser Gasstrom weiter gekühlt wird. Auch nach seinem Austritt aus dem Speicher 18 und vor seinem Eintritt in den Methanolsynthesereaktor 27 der Syntheseanlage 60 durchströmt der CO₂-reiche Gasstrom einen weiteren Wärmetauscher 20, mittels welchem in den CO₂-reichen Gasstrom Wärme eingekoppelt wird, um den als Edukt in den Methanolsynthesereaktor 27 eintretenden CO₂-reichen Gasstrom auf eine Reaktor- oder Reaktionstemperatur im Bereich von 100-400 °C, vorzugsweise von 150-300 °C, zu bringen. Die dafür benötigte Wärme wird dem Wärmetauscher 20 als Anzapfdampf, der dem Turbosatz 58 entnommen wird, oder in Form von bei anderen Prozessen entstehender Abwärme zugeführt.

In dem Methanolsynthesereaktor wird das zugeführte CO₂ des CO₂-reichen Gasstromes mit Wasserstoff zu Methanol umgesetzt. Der Wasserstoff wird in einer Elektrolyseanlage 61, bei der es sich im Ausführungsbeispiel um eine alkalische Wasserelektrolyse handelt, hergestellt. Es ist aber auch möglich anderen Elektrolyseurtypen, wie Polymerelektrolyt Membran Elektrolyseure (PEM) oder Solid Oxide Electrolyzer Cell (SOEC) oder eine Chloralkalielektrolyse anzuwenden.

Die alkalische Wasserelektrolyse des Ausführungsbeispiels umfasst einen Elektrolysezelle 21, in welcher zugeführtes Wasser 34 bei einer Temperatur zwischen 50 und 100 °C, vorzugsweise zwischen 70 und 90 °C, elektrolytisch in seine Bestandteile Wasserstoff und Sauerstoff zerlegt wird. Hierbei werden die Elektrolysezelle 21 selbst mittels eines Wärmetauschers 22b und das zugeführte Wasser mittels eine Wärmetauschers 22a temperiert, so dass die Elektrolyse sich jederzeit im optimalen Betriebstemperaturbereich befindet und schnell Laständerungen, insbesondere auch solche hin zu höheren Lasten, vollziehen kann. Die alkalische Elektrolyse kann in weiten Druckbereichen betrieben werden, wobei insbesondere Drücke oberhalb von 15 bar, vorzugsweise Drücke im Bereich von 20 bar bis 60 bar Anwendung finden. Alternativ oder zusätzlich ist die Syntheseanlage 61 mit einem Wasserstoff-Verdichter 23 ausgestattet, dem der in der Elektrolysezelle 21 erzeugte Wasserstoff vor seinem Eintritt in den Methanolsynthesereaktor 27 zu geführt wird. Ein solcher Wasserstoff-Verdichter 23 ist insbesondere deshalb zweckmäßig, weil diesem ein Wasserstoffspeicher 24 zugeordnet ist, in welchem erzeugter Wasserstoff bevorratet werden kann. Der Zwischenspeicher 24 ist einerseits ein Produktspeicher, da darin der mittels der Elektrolyseanlage 61 erzeugte Wasserstoff gespeichert wird. Er stellt andererseits aber auch einen Eduktspeicher dar, da der darin gespeicherte Wasserstoff den einen Eingangsstoff für die Methanolsynthese darstellt. Um den in dem Wasserstoff-Verdichter 23 verdichteten Wasserstoff vor seiner Zwischenspeicherung zu kühlen, ist zwischen dem Wasserstoff-Verdichter 23 und dem Zwischenspeicher 24 ein Wärmetauscher 25 angeordnet, mittels welchem Wärmeenergie aus dem Wasserstoffstrom ausgekoppelt oder entnommen werden kann. Um den später den Zwischenspeicher 24 verlassenden Wasserstoffstrom vor dessen Eintritt in den Methanolsynthesereaktor 27 auf eine ausreichend hohe Reaktionstemperatur zu bringen, ist ein weiterer Wärmetauscher 26 vorgesehen, mittels welchem in dem Wasserstoffstrom wiederum Wärme eingekoppelt wird, wobei die dazu notwendige Wärme aus aus dem Wasser/Dampf-Kreislauf 54 stammendem Anzapfdampf oder aus der Abwärme des Methanolsynthesereaktor 27 stammen kann. Im Übrigen kann die bei der Methanolsynthese entstehende Abwärme über dem Methanolsynthesereaktors 27 nachgeschaltete oder in diesen integrierte Wärmetauscher oder Kühleinrichtungen 28a, 28b, 28c abgeführt werden. Der Wärmetauscher 28a führt dabei seine Wärme an die Vorwärmstrecke des Wasser/Dampf-Kreislaufes 54 ab, wobei aber auch die Wärmeabgabe an den Reboiler 9 der Post Combustion Capture (PCC)-Anlage 5 und/oder verschiedene Eduktvorwärmungen, d.h. die Vorerwärmung in dem erfindungsgemäßen Kraftwerk zu verarbeitender/umzusetzender Ausgangsprodukte, abgeführt werden kann. Da im Methanolsynthesereaktor 27 kein sehr hoher Umsatz der zugeführten Edukte Kohlendioxid (CO₂) und Wasserstoff (H₂) erreicht wird, sondern der Umsatz lediglich im Bereich von 10-35 % liegt, ist im Ausführungsbeispiel die die Wärmetauscher/Kühleinrichtungen umfassende nachgeschaltete Kühlung 28a, 28b, 28c derart ausgelegt, dass in einem Behältnis 29 eine Phasentrennung des in dem Methanolsynthesereaktors 27 erzeugten Methanolproduktes erfolgt und die abgetrennten gasförmigen Bestandteile über eine Rückführung 30 ganz oder teilweise wieder in den Methanolsynthesereaktor 27 rückgeführt werden. In der Rückführung 30 wird dem rückgeführten gasförmigen Bestandteilen mittels des Wärmetauschers 17c wiederum Wärme zugeführt.

Die im Behältnis 29 abgetrennte flüssige Phase wird einem Destillations- oder Rektifikationsreaktor 31 zugeführt, in welchem von der flüssigen Phase Wasser, gewünschtenfalls aber auch schwer siedende Alkohole, wenn eine höhere Reinheit des zu erzeugenden Methanolproduktes gewünscht wird, abgetrennt werden. Der für die Destillation oder Rektifikation benötigte Wärmebedarf wird zweckmäßigerweise durch Wärmetauscher gedeckt, denen minderwertiger Anzapfdampf aus dem Wasser/Dampf-Kreislauf 54 und/oder aus der Abwärme des Reboilers 9 oder aber auch anderen Prozessschritten ausgekoppelte Wärmeenergie zugeführt wird. Den Destillations- und/oder Rektifikationsreaktor 31 verlässt ein gasförmiger Methanol (CH₃OH)-Strom 35, wobei insbesondere dessen Verdampfungswärme in zwei abschließenden Kühlschritten (32a, 32b) mittels Wärmetauschern 32a, 32b in die Vorwärmstrecke des Wasser/Dampf-Kreislaufes 54 und/oder eine Eduktvorwärmung ausgekoppelt werden kann.

Das aus dem Destillations- und/oder Rektifikationsreaktor 31 abgeführte Wasser 33 kann einer speziellen Wasseraufbereitungsanlage und/oder der Speisewasseraufbereitungsanlage des Wasser/Dampf-Kreislaufes 54 zugeführt und danach als Edukt (Wasser 34) der Elektrolyseanlage 61 zugeführt werden.

Der bei der Elektrolyse entstehende Sauerstoff kann komprimiert/verdichtet, gewünschtenfalls verflüssigt und einer Verwendung zugeführt werden.

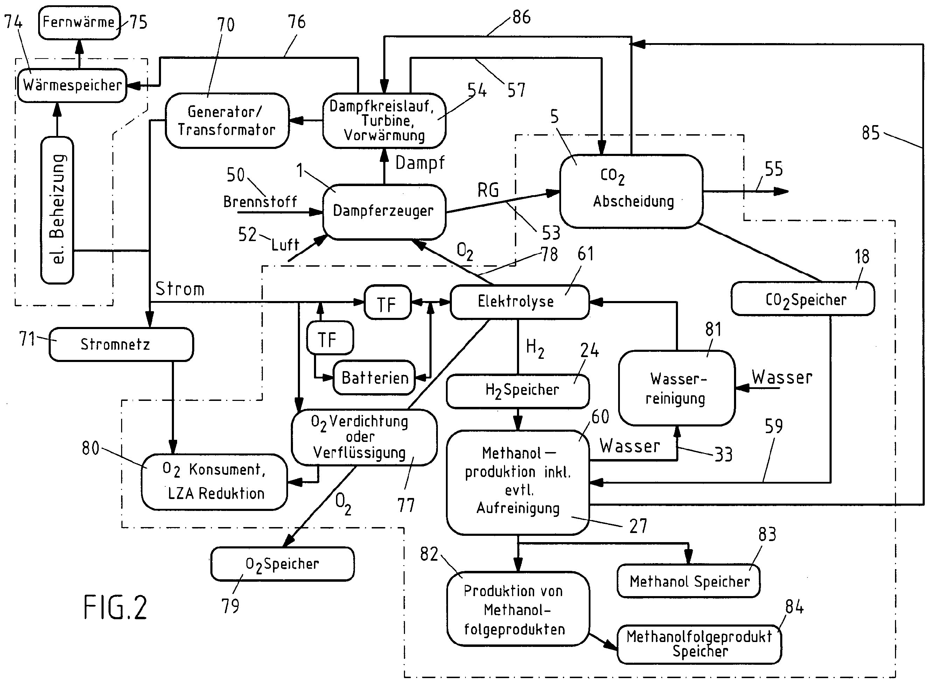

Der Fig. 2 ist schematisch die Zuordnung und Verschaltung der einzelnen Einrichtungen und Anlagen des erfindungsgemäßen Kraftwerkes zu entnehmen.

Der mittels eines Generators 70 mit gegebenenfalls zugeordnetem Transformator im Kraftwerk 51 erzeugte Strom kann einerseits in das angeschlossene öffentliche Stromnetz 71, aber auch der Elektrolyseanlage 61 zugeführt werden, wobei der Elektrolyseanlage 61 zudem Batterien 72 und Transformatoren 73 zugeordnet sind, die eine Speicherung und Transformation des zugeführten Stromes ermöglichen. Mit dem von dem Generator 70 erzeugten Strom lässt sich aber auch ein elektrisch beheizter Wärmespeicher 74 versorgen, der beispielsweise Fernwärme erzeugt, die an ein Fernwärmenetz 75 abgegeben werden kann. Dem Wärmespeicher 74 kann zudem aus dem Wasser/Dampf-Kreislauf 54 stammender Dampf 76 oder daraus ausgekoppelte Wärmeenergie zugeführt werden. Der mittels des Generators 70 erzeugte Strom kann aber auch einer Sauerstoffverdichtungs- oder verflüssigungsanlage 77 zugeführt werden, in welcher bei der Elektrolyse 61 entstehender Sauerstoff 78 verarbeitet wird. Der verdichtete oder verflüssigte Sauerstoff kann dann in einem Sauerstoffspeicher 79 gespeichert oder aber auch einer weiteren Verwendung 80 zugeführt werden. Der in der Elektrolyseanlage 61 erzeugte Sauerstoff 78 kann aber auch dem Dampferzeuger 1 als Oxidationsmittel zugeführt werden. Die einzelnen mit dem vom Generator 70 erzeugten Strom versorgten Anlagen oder Einrichtungen 61, 72, 74, 77 und 80 lassen sich aber auch - auch wenn dies in Fig. 2 nicht dargestellt ist - alle mit aus dem Stromnetz 71 bezogenem Strom, insbesondere dann, wenn dieses sogenannten Überschussstrom bereitstellt, versorgen. Insbesondere sind die dargestellten Einrichtungen/Anlagen derart miteinander verschaltet, dass der mittels des Generators 70 erzeugte oder der aus dem Stromnetz 71 bezogene Strom flexibel auf die einzelnen Einrichtungen/Anlagenteile verteilbar ist. Die Priorität liegt dabei aber auf der Methanolherstellung, insbesondere auf der Methanolherstellung mittels der Syntheseanlage 60, so dass die Elektrolyseanlage 61 im Kern die Anlage darstellt, die flexibel, zeitnah und schnell mit Laständerungen auf netzseitige Leistungsregelungsanforderungen reagiert.

Der Elektrolyseanlage 61 und der Syntheseanlage 60 ist eine Wasserreinigung 81 zugeordnet, in welcher das der Elektrolyseanlage 61 und der Syntheseanlage 60 zuzuführende Wasser vorher den gewünschten Anforderungen entsprechend gereinigt wird.

Sowohl der Methanolproduktion mittels des Methanolsynthesereaktors 27 als auch der Produktionsanlage zur Produktion von Methanolnachfolgeprodukten 82 sind jeweils Speicher zugeordnet, und zwar der Syntheseanlage 60 ein Methanolspeicher 83 und der Produktionsanlage 82 ein Methanolfolgeproduktspeicher 84. Die bei der Methanolproduktion in der Syntheseanlage 60 und die bei der CO₂-Abscheidung in der Post Combustion Capture-Anlage 5 entstehende Abwärme wird wieder in den Wasser-/Dampfkreislauf 54 eingekoppelt, wie dies durch die Pfeile 85 und 86 angedeutet ist.

Insgesamt ist das erfindungsgemäße Kraftwerk 51 durch die in den Figuren 1 und 2 dargestellten stromführenden und medienführenden Leitungsverbindungen bezüglich damit realisierbarer Betriebsweisen und einstellbarer Produktions- oder Umwandlungsleistungen oder erzeugbarer Produktions- oder Umwandlungsprodukte flexibilisiert. Auch die Stromaufnahmen oder Stromleistungsaufnahmen der einzelnen Anlagenteile, insbesondere der Elektrolyseanlage 61, tragen dazu bei, indem sie zur Flexibilisierung an unterschiedliche Leistungsanforderungen und Leistungsregelungsanforderungen anpassbare Fahrweisen oder Betriebsweisen des Kraftwerkes 51 ermöglichen. So lässt sich die CO₂-Abscheidung mittels der PCC-Anlage 5 und/oder die Wasserstoffproduktion mittels der Elektrolyseanlage 61 derart regeln, dass die Minimallast des Kraftwerkes 51 und der Stromeinspeisung in das Stromnetz 71 bis auf 0 NWₑₗ reduziert werden kann. Wenn dann zudem noch zusätzlich Strom aus dem Stromnetz 71 bezogen wird, wird die Netzeinspeisung des Kraftwerkes 51 dann insgesamt sogar negativ.

Die Elektrolyseanlage 61 kann zudem so ausgelegt sein, dass der Strombezug zur Wasserstoffherstellung um das fünf- bis zehnfache höher ist, als die jeweils aktuelle Leistung des Generators 70 im aktuellen Lastzustand des Kraftwerkes 51.

Auch kann die CO₂-Abscheideanlage 5 derart ausgelegt sein, dass bis zu 95% des mit dem Rauchgas 53 produzierten Kohlendioxids oder Kohlendioxidstroms abgeschieden wird und zeitgleich oder nach einer Zwischenspeicherung in einem CO₂-Speicher 18 zeitverzögert chemischen Reaktoren, insbesondere dem Methanolsynthesereaktors 27 zur Herstellung von Methanol oder Methanolfolgeprodukten zugeführt wird.

Die vorgesehenen Speicher CO₂-Speicher 18, Wasserstoffspeicher 24, Methanolspeicher 83, Sauerstoffspeicher 79 und Methanolfolgeproduktspeicher 84 sind als sogenannte Pufferspeicher ausgebildet, um für die diese darin gespeicherten Produkte als Edukte für die nachfolgende Weiterverarbeitung zwischenspeichern zu können. Hierbei sind die der Wasserstoffspeicher 24 und/oder der CO₂-Speicher 18 und/oder der Sauerstoffspeicher 79 vorzugsweise als Druckspeicher ausgebildet und alle Speicher mit einer Kapazität ausgestattet, die die für die Weiterverarbeitung benötigten Produktmengen speichern, so dass diese kurz- oder längerfristig gespeichert werden, aber auch gewünschtenfalls kurzfristig dem zugeordneten Produktionsprozess zur Verfügung gestellt werden können.

Weiterhin unterstützen die Elektrolyseanlage 61 und/oder die CO₂-Abscheideanlage 5 bzw. die Einrichtung zur Erzeugung eines CO₂-reichen Gasstromes und/oder die chemischen Reaktoren, insbesondere der Methanolsynthesereaktor 27 den flexiblen Betrieb des Kraftwerkes durch die Bereitstellung einer schnellen Laständerung derart, dass die gesamte Regelfähigkeit, insbesondere die Primär- und Sekundärregelung des Kraftwerkes in Bezug auf eine Stromeinspeisung oder einen Strombezug verbessert wird. Die von diesen Anlagen, insbesondere aber das der Elektrolyseanlage 61 bereitgestellte Laständerungsvermögen führt dazu, dass das Kraftwerk 51 dann mit einem Laständerungsgradienten von 3%/m bis 10 bis über 20%/m betrieben werden kann. Natürlich ist es auch möglich, in dem Kraftwerk zusätzlich noch Anlagen vorzusehen, die erneuerbare Energie erzeugen, wie Photovoltaikanlagen oder Windkraftanlagen, die dann ebenfalls den erzeugten Strom dem Gesamtprozess zur Verfügung stellen.

Die bei den einzelnen Prozessen in den aufgeführten Anlagen jeweils entstehende oder benötigte Prozesswärme kann durch entsprechende Leitungsverschaltung der einzelnen Anlagen oder Teilprozesse bereitgestellt werden. So ist es möglich, Prozesswärme, insbesondere im Bereich von 30 bis 150°C, die als Abwärme aus der Post Combustion Capture-Anlage 5 und/oder als Anzapfdampf aus dem Wasser/Dampf-Kreislauf 54 entnommen wird, zur Vorwärmung von Prozessströmen eines Elektrolyseurs, insbesondere der Elektrolyseanlage 61, und/oder zur Begleitheizung desselben/derselben und/oder zur Vorwärmung von in dem Methanolsynthesereaktor 27 umzusetzenden Edukten und/oder zur Speisewasservorwärmung im Wasser/Dampf-Kreislauf 54 und/oder in dem Destillations- und/oder Rektifikationsreaktor 31 eingesetzt wird. Auch kann Abwärme, die aus der Kühlung von Produkten in der Eduktvorwärmung der Elektrolyse, aus Reaktoren oder der Aufreinigung von Produkten stammt, dem Gesamtprozess wieder zugeführt werden. Auch kann das bei der Destillation oder der Rektifikation gewonnene Wasser 33 oder gegebenenfalls an anderer Stelle des Gesamtprozesses gewonnenes Wasser vorzugsweise nach Behandlung in einer Wasserreinigung 81 wieder der Elektrolyseanlage 61 zugeführt werden. Der bei der Elektrolyse von Wasser entstehende Sauerstoff kann zumindest teilweise einem benachbarten Industriebetrieb zugeführt und dort die Auslastung von Luftzerlegungsanlagen reduzieren. Es ist aber auch möglich, den bei der Elektrolyse von Wasser entstehenden Sauerstoff zumindest zum Teil zu verdichten und in Druckbehälter oder zumindest einen Sauerstoffspeicher 79 abzufüllen und/oder durch einen Kälteprozess zu verflüssigen.

Die Wasserstoffproduktion und/oder andere Anlagenteile, insbesondere elektrische Anlagenteile, sind derart dimensioniert, dass zumindest kurzzeitig im Minutenbereich, vorzugsweise bis zu über 30 Minuten, der elektrische Verbrauch auf über 100% des Auslegungswertes, vorzugsweise bis über 120 bis 200%, erhöht werden kann. Ferner sind die zusätzlich installierten Batterien 72 derart dimensioniert, dass zumindest kurzzeitig im Sekundenbereich, vorzugsweise auch bis zu über 15 Minuten, der elektrische Verbrauch oder die elektrische Einspeisung auf über 100% des Auslegungswertes, vorzugsweise bis über 150 bis 300% erhöht werden kann.

Weiterhin kann vorgesehen sein, dass eine elektrische Wassererhitzung und/oder Dampferzeugung im Gesamtprozess bzw. im Kraftwerk 51 installiert ist, die einerseits im Bezug auf ihre Stromaufnahme zur Flexibilisierung des Kraftwerkes beiträgt und deren erzeugte Wärme (warmes Wasser oder Dampf) einem oder mehreren Wärmespeichern zuführbar ist. Insbesondere ist im Kraftwerk daher auch vorgesehen, dass eine Wärmespeicherung in Form von Wasser, Dampf oder Feststoffen oder Flüssigkeiten wie Salzen integriert ist. Auch kann die erzeugte oder gespeicherte Wärme für die Trocknung des eingesetzten Brennstoffes, insbesondere der beim Ausführungsbeispiel vorgesehenen Braunkohle, genutzt werden, indem Abwärme aus den Teilprozessen oder einer Turbinenanzapfung (Turbosatz) zur Trocknung von Braunkohle oder anderen Brennstoffen eingesetzt wird.

In der Figur 1 sind folgende Abkürzungen enthalten: GP = Gasphase, FP = Flüssigphase, HDV = Hochdruckvorwärmung, NDV = Niederdruckvorwärmung, HD = Hochdruckturbine, MD = Mitteldruckturbine und ND = Niederdruckturbine.

Schließlich kann auch vorgesehen sein, dass ein Teil der insbesondere mit der Syntheseanlage 60 erzeugten Reaktionsprodukte, d. h. Methanol oder ein Methanolfolgeprodukt am Kraftwerksstandort gelagert und als Startbrennstoff und/oder als Unterstützungsbrennstoff und/oder als Hauptbrennstoff zumindest zeitweise den Brennern des Dampferzeugers 1 zugeführt und dort verbrannt wird. Unter einem CO_{2'}-reichen Gasstrom wird im Rahmen dieser Anmeldung ein solcher verstanden, der einen Anteil von mindesten 12, insbesondere mindestens 30, Gewichtsprozent oder Volumenprozent an CO₂ aufweist.

Zusammenfassend lässt sich feststellen, dass die Erfindung von der Überlegung ausgeht, dass sich bei Kraftwerken, die ansonsten heruntergefahren werden müssten, durch die Erhöhung des Eigenbedarfes an Energie mittels der CO₂-Abscheidung 5 (insbesondere Bedarf an Dampf für die Erwärmung des Reboilers 9) und mittels einer Methanolherstellung durch eine Syntheseanlage 60 (Bedarf an Strom für die Elektrolyse) die Ausnutzung der vorhandenen Kraftwerkskapazitäten dadurch erhöhen lässt, dass auch in Zeiten schwacher Nachfrage das Kraftwerk mit einer höheren Last betrieben werden kann, als es für die reine Stromerzeugung notwendig wäre. Im Endergebnis kann das Kraftwerk dann mit einer höheren Anzahl an Volllaststunden betrieben werden, da es eben nicht nur zur Stromproduktion, sondern auch zur Methanolproduktion oder der Produktion von Methanolfolgeprodukten ausgelegt ist. Weiterhin liegt der Erfindung die Überlegung zugrunde, dass sich die gegenüber der möglichen Laständerungsgeschwindigkeit des Kraftwerkes höhere Laständerungsgeschwindigkeit der Elektrolyseanlage 61 dazu nutzen lässt, dem angeschlossenen öffentlichen Stromnetz gegenüber einen deutlich schnelleren Regelservice zu bieten. Bei einer stromnetzseitig gesteuerten oder angeforderten Leistungsregelung kann die Elektrolyseanlage 41 relativ schnell und kurzfristig heraufgefahren oder heruntergefahren werden, so dass damit dem Dampferzeuger bzw. dem Kraftwerk insgesamt mehr Zeit gegeben wird, eine Laständerung vorzunehmen, oder sogar vermieden wird, dass eine Laständerung des eigentlichen Kraftwerksteils, d. h. eine Leistungsanpassung des Dampferzeugers 1, notwendig wird. Schließlich geht die Erfindung von der Überlegung aus, dass es mit der Erfindung möglich ist, zu vermeiden, dass in nachfrageschwachen Zeiten das Kraftwerk gänzlich abgeschaltet werden muss. Es kann immer noch so ausgelegt werden, dass die Elektrolyseanlage 61 und die Methanolproduktion 60 bei Minimallast des Kraftwerkes ausreichend Strom beziehen bzw. abnehmen und keine Stromabgabe ins Netz notwendig wird. Grenzfälle der erfindungsgemäßen Auslegung eines Kraftwerkes sind einerseits Braunkohlekraftwerke, die derzeit (noch) sehr kostengünstig elektrische Leistung erzeugen. Ein solches Kraftwerk kann mit der Erfindung immer auf 100% Last (Volllast) gefahren werden, auch wenn das Stromnetz die erzeugte elektrische Leistung in Form von Strom nicht abnimmt. Der nicht abgenommene Strom kann in der Elektrolyseanlage 61 zur Erzeugung von Wasserstoff benutzt werden. Der andere Grenzfall sind Steinkohlekraftwerke, die heutzutage mit einer Minimallast gefahren werden müssen, da sie ansonsten eine zu lange Zeit für ein Wiederanfahren benötigen würden oder die für Regelungszwecke eine spezielle Generatorleistung zur Verfügung halten müssen. Bei erfindungsgemäß ausgestatteten Steinkohlekraftwerken kann die Minimallast nun (über)absorbiert und der erzeugte Strom für die Wasserstofferzeugung in der Elektrolyseanlage 61 genutzt werden, ohne dass das Kraftwerk vom Netz genommen werden muss, so dass dem Netz dessen träge, rotierende Masse (insbesondere die des Generators) weiterhin zur Regelunterstützung zu Verfügung steht.

Insofern wird eine Flexibilisierung der Betriebsweise eines Kraftwerkes ermöglicht, da das eigentliche Kraftwerk 51 seine Last weiterhin langsam ändern kann, aber die durch die Elektrolyseanlage 61 und die Syntheseanlage 60 genutzte Last zur Regelung zur Verfügung steht.

Insgesamt lässt sich durch die vorstehend erwähnten Regelungsmöglichkeiten die Wirtschaftlichkeit eines Kraftwerkes verbessern (mehr Volllaststunden, immer zur Verfügung gestellte Netzdienstleistungen, zusätzliches Produkt "Brennstoff" (Methanol und Methanolfolgeprodukte)).

Von Vorteil ist es bei der vorliegenden Erfindung, dass eine vorhergehende Vergasung von Produkten zur Herstellung von Methanol oder Methanolfolgeprodukten nicht erfolgen muss, sondern hierfür die Verbrennung von kohlenstoffhaltigem Brennstoff im Dampferzeuger 1 eines/des Kraftwerkes 51 genutzt wird. Die Erfindung zeichnet sich durch geringe Investitionskosten bei der Nachrüstung bestehender Kraftwerke, die Erhöhung der Wirtschaftlichkeit bestehender Anlagen bei einer Nachrüstung sowie eine hohe Betriebssicherheit und Zuverlässigkeit der Methanolsynthese aus, die bei der Verbrennung im Dampferzeuger 1 entstehendes CO₂ und durch eine Elektrolyse erzeugtes H₂ verwendet. Auch bei Neubauten stellt die erfindungsgemäße Lösung eine Verbesserung der Flexibilität, Anlagenausnutzungszeit und Wirtschaftlichkeit dar.

In dem in Fig. 1 schematisch dargestellten Kraftwerk werden beispielsweise beim Betrieb eines 670 MWel Kraftwerkes bei 30% Last 45 kg/s Brennstoff (Heizwert 10,5 MJ/kg) benötigt, 190 Mel Strom erzeugt, 15% des im Rauchgas enthaltenen CO₂ abgeschieden und 1,1 kg/s Wasserstoff elektrolytisch erzeugt. Daraus werden ca. 6 kg/s Methanol erzeugt, was einer Kohlenstoffumwandlungseffizienz vom Brennstoff zu dem Produkt Methanol von ca. 27% und einem Wirkungsgrad von über 60% (Strom zu Heizwert des Methanols) entspricht.

Würden diese Produktmengen jeweils an 90% der Jahresbetriebsstunden eines solchen Kraftwerks erzeugt, bei unterstellten Brennstoffkosten in Höhe von 10 €/t sowie Erlösen von 400 €/t Methanol, ließe sich ein Umsatz von 60Mio Euro erzielen. Weiterhin kann das Kraftwerk zusätzliche Erlöse durch eine höhere Stromproduktion, eine nahezu ganzjährige Primär/Sekundärregelung generieren. Schließlich sind auch Erlöse durch Demandside-Management mittels der Elektrolyseanlage 61 möglich. Insbesondere kann die Elektrolyseanlage 61 selbst wesentlich schneller als das Kraftwerk jederzeit Lasten zwischen 0 und bis über 200 MW, gegebenenfalls bis 400 MW, (Überlasten nur kurzzeitig) fahren, so dass zusätzliche Netzdienstleistungen erbracht werden können.

Hieraus ergibt sich, dass die erfindungsgemäße Flexibilisierung des Kraftwerks 51 technisch wie wirtschaftlich einen positiven Einfluss auf den Betrieb des Kraftwerkes 51 hat.

## Patentansprüche

1. Kraftwerk (51), das einen mit kohlenstoffbefeuerten Brennern und/oder einer Gasturbine ausgestatteten Großdampferzeuger (1) mit angeschlossenem Wasser/Dampfkreislauf (54) aufweist, der mindestens einen dampfbeaufschlagten Turbosatz (58) mit mindestens einem angeschlossenen Generator (70) umfasst, wobei in dem mit den kohlenstoffbefeuerten Brennern ausgestatteten Großdampferzeuger (1) ein CO₂-haltiger Abgasstrom (53) erzeugt wird,
und das mindestens eine Einrichtung zur Erzeugung eines CO₂-reichen Gasstroms umfasst,
und das mit seinem den mindestens einen Generator (70) umfassenden stromerzeugenden Teil an ein öffentliches Stromnetz (71) angeschlossen ist, das Regelleistung bereitstellt, wobei die elektrische Leistungsabgabe des stromerzeugenden Teils des Kraftwerks (51) an das Stromnetz (71) einer stromnetzseitig gesteuerten Leistungsregelung, insbesondere einer Primärregelung und/oder Sekundärregelung und/oder Tertiärregelung und/oder Quartärregelung, unterliegt,
**dadurch gekennzeichnet,**
**dass** das Kraftwerk (51) mindestens eine Elektrolyseanlage (61) zur Herstellung von Wasserstoff (H₂) und mindestens eine Syntheseanlage (60) zur Herstellung von Methanol und/oder Methanolfolgeprodukten aus zumindest CO₂-Anteilen des CO₂-reichen Gasstroms und des in der Elektrolyseanlage (61) erzeugten Wasserstoffs umfasst und, dass die mindestens eine Einrichtung zur Erzeugung eines CO₂-reichen Gasstroms und die mindestens eine Elektrolyseanlage (61) zur Herstellung von Wasserstoff (H₂) und die mindestens eine Syntheseanlage (60) zur Herstellung von Methanol und/oder Methanolfolgeprodukten aus zumindest CO₂-Anteilen des CO₂-reichen Gasstroms und des in der Elektrolyseanlage (61) erzeugten Wasserstoffs mittels stromführender und mittels medienführender Leitungen derart leitungsmäßig miteinander verbunden und verschaltet sind, dass der beim Betrieb des Kraftwerks (51) kraftwerksseitig erzeugte Strom ganz oder teilweise wahlweise zum Betrieb einer, mehrerer oder aller dieser aus der mindestens einen Einrichtung zur Erzeugung eines CO₂-reichen Gasstroms, der mindestens einen Elektrolyseanlage (61) zur Herstellung von Wasserstoff (H₂) und der mindestens einen Syntheseanlage (60) zur Herstellung von Methanol und/oder Methanolfolgeprodukten bestehenden Gruppe an Einrichtungen und Anlagen nutzbar ist.

2. Kraftwerk nach Anspruch 1, **dadurch gekennzeichnet, dass** die mindestens eine Elektrolyseanlage (61) zur Herstellung von Wasserstoff (H₂) oder mehrere Elektrolyseanlagen (61) zur Herstellung von Wasserstoff (H₂) und/oder die mindestens eine Einrichtung zur Erzeugung eines CO₂-reichen Gasstroms oder mehrere Einrichtungen zur Erzeugung eines CO₂-reichen Gasstroms und/oder die mindestens eine Syntheseanlage (60) zur Herstellung von Methanol und/oder Methanolfolgeprodukten aus zumindest Teilen des CO₂-reichen Gasstroms oder mehrere Syntheseanlagen (60) zur Herstellung von Methanol und/oder Methanolfolgeprodukten aus zumindest Teilen des CO₂-reichen Gasstroms kraftwerksseitig bezüglich ihres Strom- oder Leistungsaufnahmevermögens und ihrer Wasserstofferzeugungskapazität oder hinsichtlich ihres Strom- oder Leistungsaufnahmevermögens und ihrer Produktions- oder Umwandlungsleistung derart ausgelegt und derart regelbar eingerichtet ist/sind, dass ihre Strom- oder Leistungsaufnahme und Wasserstofferzeugung und/oder ihre jeweilige Strom- oder Leistungsaufnahme und Produktions- oder Umwandlungsleistung in Antwort auf eine netzseitige Leistungsregelungsanforderung an das Kraftwerk (51) kurzfristig, vorzugsweise im Minutenbereich, hoch- oder herunterfahrbar ist.

3. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms kraftwerksseitig bezüglich ihres jeweiligen Strom- oder Leistungsaufnahmevermögens und ihrer jeweiligen Produktions- oder Umwandlungsleistung derart ausgelegt und regelungstechnisch miteinander verbunden sind, dass sie in Antwort auf eine netzseitige Leistungsregelungsanforderung an das Kraftwerk (51) im Verbund jeweils bezüglich ihrer jeweiligen Strom- oder Leistungsaufnahme und Produktions- oder Umwandlungsleistung derart kurzfristig, vorzugsweise im Minutenbereich, hoch- oder herunterfahrbar sind, dass das Kraftwerk (51) im Falle einer netzseitigen Leistungsregelungsanforderung leistungsmäßig im Wege einer Laständerung mit einem Laständerungsgradient im Bereich von 3%/min - 30%/min an die geänderte Leistungsanforderung anpassbar ist.

4. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms bezüglich ihrer jeweiligen Strom- oder Leistungsaufnahme und ihrer jeweiligen Produktions- oder Umwandlungsleistung individuell ansteuerbar und regelbar ausgebildet sind und /oder bezüglich ihrer jeweiligen Strom- oder Leistungsaufnahme und/oder ihrer jeweiligen Produktions- oder Umwandlungsleistung derart ausgelegt ist/sind, dass sie, insbesondere in Antwort auf eine netzseitige Leistungsregelungsanforderung an das Kraftwerk (51), kurzzeitig im Minutenbereich, vorzugsweise über einen Zeitraum von bis zu 30 Minuten, mit einer Strom- oder Leistungsaufnahme beaufschlagbar ist/sind, die 100 - 300 %, vorzugsweise 150 - 200 %, des Normalauslegungs- oder Normalbetriebswertes der jeweiligen Anlage (60, 61) oder Einrichtung beträgt.

5. Kraftwerk nach einem der vorhergehenden Ansprüche, **dadurch gekennzeichnet, dass** die mindestens eine Syntheseanlage (60) zur Herstellung von Methanol und/oder Methanolfolgeprodukten oder die mehreren Syntheseanlagen (60) zur Herstellung von Methanol und/oder Methanolfolgeprodukten insgesamt kapazitätsmäßig derart ausgelegt ist/sind, dass damit 10 - 50 Gew.-%, insbesondere 30 - 40 Gew.-%, bevorzugt 35 Gew.-%, des bei Volllast des Kraftwerks (51) entstehenden und in dem CO₂-haltigen Abgasstrom (53) enthaltenen CO₂ zu Methanol und/oder einem Methanolfolgeprodukt umwandelbar ist und/oder insgesamt bezüglich ihres Strom- oder Leistungsaufnahmevermögens und der jeweils möglichen Produktions- oder Umwandlungsleistung kapazitätsmäßig derart ausgelegt ist/sind, dass maximal der bei Volllast und/oder Maximalleistung des Kraftwerks (51) von diesem insgesamt erzeugbare elektrische Strom für die Herstellung von Methanol und/oder Methanolfolgeprodukte nutzbar ist.

6. Kraftwerk nach einem der vorhergehenden Ansprüche, **dadurch gekennzeichnet, dass** die mindestens eine Einrichtung zur Erzeugung eines CO₂-reichen Gasstroms oder die mehreren Einrichtungen zur Erzeugung eines CO₂-reichen Gasstroms mindestens eine CO₂-Abscheideanlage, insbesondere eine Post Combustion Capture (PCC)-Anlage (5) und/oder einen oder mehrere nach dem Oxyfuel-Prozess betriebene(n) Brenner oder Brennereinrichtungen des Großdampferzeugers (1) mit zugeordneter CO₂-Abscheideanlage, umfasst/umfassen oder aus diesen besteht/bestehen.

7. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms bezüglich ihrer Strom- oder Leistungsaufnahme und Produktions- oder Umwandlungsleistung insgesamt derart ausgelegt sind, dass bei ihrem Betrieb das Kraftwerk (51) bei einem Betrieb mit seiner anlagentechnisch notwendigen Minimallast ohne Stromeinspeisung in das Stromnetz (71) betreibbar ist.

8. Kraftwerk nach einem der vorhergehenden Ansprüche, **dadurch gekennzeichnet, dass** das Kraftwerk (51) als Stromsenke für das angeschlossenen öffentliche Stromnetz (71) ausgebildet ist, wobei die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms bezüglich ihrer Strom- oder Leistungsaufnahme und Produktions- oder Umwandlungsleistung insgesamt derart ausgelegt und mit dem Stromnetz (71) verschaltet sind, dass sie mit aus dem Stromnetz (71) bezogenem Überschussstrom betreibbar sind.

9. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms als abschaltbare Last mit dem öffentlichen Stromnetz (71) verbunden und verschaltet sind.

10. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten in einer beim Betrieb dieser Anlage(n) (60, 61) und/oder Einrichtung(en) im Bereich von 30 - 400°C, vorzugsweise im Bereich von 30 - 150°C, entstehende Abwärme über mindestens eine Abwärme führende Leitung mit einer Vorwärmung des Speisewassers des Wasser/Dampfkreislaufs (54) und/oder einer Vorwärmung einer CO₂-Abscheideanlage, insbesondere einer Post Combustion Capture (PCC) - Anlage (5), und/oder einer Vorwärmung mindestens eines der in dem Kraftwerk (51) eingesetzten in Edukte und/oder erzeugten Produkte in Leitungsverbindung steht.

11. Kraftwerk nach einem der vorhergehenden Ansprüche, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) bezüglich ihrer Produktions- und/oder Umwandlungskapazität derart ausgelegt ist/sind, dass mit der erzeugbaren Wasserstoffmenge der gesamte CO₂-Anteil des bei der Verbrennung kohlenstoffhaltigen Brennstoffs (50) in den Brennern des Großdampferzeugers (1) entstehenden Abgasstroms (53) und/oder das gesamte in der mindestens einen CO₂-Abscheideanlage (5) abgeschiedene CO₂ in der oder den Syntheseanlagen (60) zur Herstellung von Methanol und/oder Methanolfolgeprodukten zu Methanol oder einem Methanolfolgeprodukt umsetzbar ist.

12. Kraftwerk nach einem der vorhergehenden Ansprüche, **dadurch gekennzeichnet, dass** jeder der Einrichtungen oder Anlagen aus der Gruppe der mindestens einen Einrichtung zur Erzeugung eines CO₂-reichen Gasstroms, der mindestens einen Elektrolyseanlage (61) zur Herstellung von Wasserstoff (H₂) und der mindestens Syntheseanlage (60) zur Herstellung von Methanol und/oder Methanolfolgeprodukten mindestens ein Edukt- und/oder Produktspeicher, insbesondere der Elektrolyseanlage (61) ein Wasserstoffspeicher (24) und/oder ein Sauerstoffspeicher (79) und der Einrichtung zur Erzeugung eines CO₂-reichen Gasstroms ein CO₂-Speicher (18), zugeordnet ist.

13. Verfahren zum flexiblen Betrieb eines Kraftwerks (51) nach einem der Ansprüche 1 - 12, **dadurch gekennzeichnet, dass** die mindestens eine Einrichtung zur Erzeugung eines CO₂-reichen Gasstroms und die mindestens eine Elektrolyseanlage (61) zur Herstellung von Wasserstoff (H₂) und die mindestens eine Syntheseanlage (60) zur Herstellung von Methanol und/oder Methanolfolgeprodukten aus zumindest CO₂-Anteilen des CO₂-reichen Gasstroms und des in der Elektrolyseanlage (61) erzeugten Wasserstoffs mit stromführenden und medienführenden Leitungen leitungsmäßig miteinander verbunden und verschaltet sind und werden, so dass der beim Betrieb des Kraftwerks (51) kraftwerksseitig erzeugte Strom ganz oder teilweise wahlweise zum Betrieb einer, mehrerer oder aller dieser aus der Einrichtung zur Erzeugung eines CO₂-reichen Gasstroms, der Elektrolyseanlage (61) zur Herstellung von Wasserstoff (H₂) und der Syntheseanlage (60) zur Herstellung von Methanol und/oder Methanolfolgeprodukten bestehenden Gruppe an Einrichtungen und Anlagen genutzt wird.

14. Verfahren nach Anspruch 13, **dadurch gekennzeichnet, dass** die Strom- oder Leistungsaufnahme und die Wasserstofferzeugung der mindestens einen Elektrolyseanlage (61) zur Herstellung von Wasserstoff (H₂) oder der mehreren Elektrolyseanlagen (61) zur Herstellung von Wasserstoff (H₂) kraftwerksseitig in Antwort auf eine netzseitige Leistungsregelungsanforderung an das Kraftwerk (51) kurzfristig, vorzugsweise im Minutenbereich, hoch oder herunter gefahren wird.

15. Verfahren nach Anspruch 13 oder 14, **dadurch gekennzeichnet, dass** die jeweilige Strom-/Leistungsaufnahme und Produktions- oder Umwandlungsleistung der mindestens einen Einrichtung zur Erzeugung eines CO₂-reichen Gasstroms oder mehrerer Einrichtungen zur Erzeugung eines CO₂-reichen Gasstroms und/oder der mindestens einen Syntheseanlage (60) zur Herstellung von Methanol und/oder Methanolfolgeprodukten aus zumindest Teilen des CO₂-reichen Gasstroms oder mehrerer Syntheseanlagen (60) zur Herstellung von Methanol und/oder Methanolfolgeprodukten aus zumindest Teilen des CO₂-reichen Gasstroms kraftwerksseitig in Antwort auf eine netzseitige Leistungsregelungsanforderung an das Kraftwerk (51) kurzfristig, vorzugsweise im Minutenbereich, hoch oder heruntergefahren wird.

16. Verfahren nach einem der Ansprüche 13 bis 15, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms bezüglich ihrer jeweiligen Strom- oder Leistungsaufnahme und ihrer jeweiligen Produktions- oder Umwandlungsleistung individuell angesteuert und geregelt werden.

17. Verfahren nach einem der Ansprüche 13 bis 16, **dadurch gekennzeichnet, dass** die mindestens eine Elektrolyseanlage (61) zur Herstellung von Wasserstoff (H₂) oder die mehreren Elektrolyseanlagen (61) zur Herstellung von Wasserstoff (H₂) und die mindestens eine Einrichtung zur Erzeugung eines CO₂-reichen Gasstroms oder die mehreren Einrichtungen zur Erzeugung eines CO₂-reichen Gasstroms und die mindestens eine Syntheseanlage (60) zur Herstellung von Methanol und/oder Methanolfolgeprodukten aus zumindest Teilen des CO₂-reichen Gasstroms oder die mehreren Syntheseanlagen (60) zur Herstellung von Methanol und/oder Methanolfolgeprodukten aus zumindest Teilen des CO₂-reichen Gasstroms als mit dem öffentlichen Stromnetz (71) verbundene und verschaltete abschaltbare Last betrieben werden.

## Claims

1. Power plant (51), having a utility steam generator (1) equipped with burners fired by carbon-containing fuel and/or with a gas turbine and connected to a water/steam circuit (54), said generator comprising at least one steam-driven turbine set (58) with at least one connected generator (70), wherein a flue gas flow (53) containing CO₂ is generated in the utility steam generator (1) equipped with the burners fired by carbon-containing fuel, and that said plant comprises at least one device for generating a CO₂-rich gas flow.
and that said plant is connected to a public electrical grid (71) with its electricity-generating part comprising the at least one generator (70) and providing the controlling power wherein the electrical power output of the electricity-generating part of the power plant (51) to the electrical grid (71) is subject to power adaptation controlled by the power grid side, in particular to primary adaptation and/or secondary adaptation and/or tertiary adaptation and/or quaternary adaptation,
**characterised in that**
the power plant (51) comprises at least one electrolysis installation (61) for producing hydrogen (H₂) and at least one synthesis installation (60) for producing methanol and/or secondary products of methanol from at least CO₂ fractions of the CO₂-rich gas flow and of the hydrogen produced in the electrolysis installation (61) and that the at least one device for producing a CO₂-rich gas flow and the at least one electrolysis installation (61) for producing hydrogen (H₂) and the at least one synthesis installation (60) for producing methanol and/or secondary products of methanol from at least CO₂ fractions of the CO₂-rich gas flow and of the hydrogen produced in the electrolysis installation (61) are connected to and interconnected with each other in terms of circuitry by means of current-conducting and media-conducting lines in such a way that the electricity generated by the power plant while the power plant (51) is operating can be utilised optionally partially or entirely for the operation of one, more or all of this group of devices and installations consisting of the at least one device for generating a CO₂-rich gas flow, the at least one electrolysis installation (61) for producing hydrogen (H₂) and the at least one synthesis installation (60) for producing methanol and/or secondary products of methanol.

2. Power plant according to claim 1, **characterised in that** the at least one electrolysis installation (61) for producing hydrogen (H₂) or a plurality of electrolysis installations (61) for producing hydrogen (H₂) and/or the at least one device for generating a CO₂-rich gas flow or a plurality of devices for generating a CO₂-rich gas flow and/or the at least one synthesis installation (60) for producing methanol and/or secondary products of methanol from at least fractions of the CO₂-rich gas flow or a plurality of synthesis installations (60) for producing methanol and/or secondary products of methanol from at least fractions of the CO₂-rich gas flow with regard to its/their respective current or power consumption capacity and its/their hydrogen production capacity or with regard to its/their current or power consumption capacity and its/their production or conversion performance is/are designed and configured in the power plant in terms of control in such a way that its/their current or power consumption and hydrogen generation can be run up or run down in a short time, preferably in a matter of minutes, in response to a power adaptation demand on the power plant (51) from the electricity grid.

3. Power plant according to either one of the preceding claims, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow with regard to their respective current or power consumption capacity and their respective production or conversion performance are designed and controllably connected in the power plant to each other in such a way that they can be run up or run down in a short time, preferably in a matter of minutes, in response to a power adaptation demand on the power plant (51) from the electricity grid linked in each case to their respective current or power consumption capacity and their production or conversion performance, and that the power plant (51) is adaptable to the changed power demand in the case of a power regulation demand from the electricity grid wherein the rate of change in power load can be in the range of 3% / min - 30% / min.

4. Power plant according to any one of the preceding claims, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow with regard to their respective current or power consumption capacity and their respective production or conversion performance are designed to be individually controllable and adjustable and/or with regard to their respective current or power consumption capacity and/or their respective production or conversion performance is/are designed, in particular in response to a power adaptation demand on the power plant (51) from the electricity grid, in such a way that it/they are subjected to a current or power consumption capacity requirement in a short time in a matter of minutes, preferably in a time up to 30 minutes, wherein said consumption requirement is 100 - 300 %, preferably 150 - 200 %, of the normal design or normal operating value of the particular plant (60, 61) or device.

5. Power plant according to any one of the preceding claims, **characterised in that** the at least one synthesis installation (60) for producing methanol and/or secondary products of methanol or the plurality of synthesis installations (60) for producing methanol and/or secondary products of methanol is/are designed in overall capacity terms so that 10 - 50 %w/w, in particular 30 - 40 %w/w, preferably 35 %w/w of the CO₂ occurring with the plant (51) at full load and contained in the CO₂-rich flue gas flow (53) can be converted into methanol and/or a secondary product of methanol and/or overall with regard to its/their current or power consumption capacity and the respective potential production or conversion performance are designed in terms of capacity such that the maximum amount of electrical current that can be generated by the plant at full load and/or at maximum power of the plant (51) can be utilised for the production of methanol and/or secondary products of methanol.

6. Power plant according to any one of the preceding claims, **characterised in that** the at least one device for generating a CO₂-rich gas flow or the plurality of devices for generating a CO₂-rich gas flow comprise(s) or consist(s) of at least one CO₂ separation system, in particular a post combustion capture (PCC) installation (5) and/or one or a plurality of burners or burner devices, operated according to the oxy fuel process, of the utility steam generator (1) with an associated CO₂ separation system.

7. Power plant according to any one of the preceding claims, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow with regard to their current or power consumption capacity and their production or conversion performance are designed overall such that, as they are operating, the power plant (51) can be operated at its technically lowest load level without supplying electricity to the power grid (71).

8. Power plant according to any one of the preceding claims, **characterised in that** the power plant (51) is designed as a current sink for the connected public power grid (71) wherein the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow with regard to their current or power consumption capacity and their production or conversion performance are designed overall and interconnected with the power grid (71) in such a way that they can be operated with surplus electricity obtained from the power grid (71).

9. Power plant according to any one of the preceding claims, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow are connected to and interconnected with the public power grid (71) as an interruptible load.

10. Power plant according to any one of the preceding claims, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow in waste heat in the range of 30 - 400°C, preferably in the range of 30 - 150°C, occurring while this/these installation(s) (60, 61) and/or device(s) is/are operating, is/are connected, via at least one line conducting waste heat, to a preheater of the feed water of the water/steam circuit (54) and/or to a preheater of a CO₂ separation system, in particular to a post combustion capture (PCC) system (5), and/or to a preheater of at least one of the educts and/or generated products used in the power plant (51).

11. Power plant according to any one of the preceding claims, **characterised in that** the at least one electrolysis installation (61) for producing hydrogen (H₂) or the plurality of electrolysis installations (61) for producing hydrogen (H₂), with regard to their production and/or conversion capacity is/are designed such that, with the generated hydrogen content, the entire CO₂ fraction of the flue gas flow (53) occurring during the combustion of carboniferous fuels (50) in the burners of the utility steam generator (1) and/or the entire CO₂ separated in the at least one CO₂ separation system (5) in the synthesis installation(s) (60) for producing methanol and/or secondary products of methanol can be converted into methanol or into one secondary product of methanol.

12. Power plant according to any one of the preceding claims, **characterised in that** an educt and/or a product storage device is assigned to each of the devices or installations from the group consisting of the at least one device for generating a CO₂-rich gas flow, the at least one electrolysis installation (61) for producing hydrogen (H₂) and the at least one synthesis installation (60) for producing methanol and/or secondary products of methanol, wherein, in particular, a hydrogen storage device (24) and/or an oxygen storage device (79) is/are assigned to the electrolysis installation (61) and a CO₂ storage device (18) is assigned to the device for generating a CO₂-rich gas flow.

13. Method for the flexible operation of a power plant (51) according to any one of claims 1 - 12, **characterised in that** the at least one device for generating a CO₂-rich gas flow and the at least one electrolysis installation (61) for producing hydrogen (H₂) and the at least one synthesis installation (60) for producing methanol and/or secondary products of methanol from at least CO₂ fractions of the CO₂-rich gas flow and of the hydrogen generated in the electrolysis installation (61) are connected and interconnected with each of them in terms of circuitry by current- and media-conducting lines in such a way that the current generated by the power plant (51) during its operation is used optionally partially or completely to operate one, more or all of this group of devices and installations consisting of the device for generating a CO₂-rich gas flow, the electrolysis installation (61) for producing hydrogen (H₂) and the synthesis installation (60) for producing methanol and/or secondary products of methanol.

14. Method according to claim 13, **characterised in that** the current or power consumption and the hydrogen generation of the at least one electrolysis installation (61) for producing hydrogen (H₂) or the plurality of electrolysis installations (61) for producing hydrogen (H₂) is/are run up or run down in a short time, preferably in a matter of minutes, in response to a power regulation/adjustment demand on the power plant (51) from the electricity grid.

15. Method according to claim 13 or 14, **characterised in that** the respective current / power consumption capacity and production or conversion performance of the at least one device for generating a CO₂-rich gas flow or the plurality of devices for generating a CO₂-rich gas flow and/or the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow is/are run up or run down in a short time, preferably in a matter of minutes, in response to a power adaptation demand on the power plant (51) from the electricity grid.

16. Method according to any one of claims 13 to 15, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow with regard to their respective current or power consumption capacity and their respective production or conversion performance are individually controlled and regulated.

17. Method according to any one of claims 13 to 16, **characterised in that** the at least one electrolysis installation (61) for producing hydrogen (H₂) or the plurality of electrolysis installations (61) for producing hydrogen (H₂) and the at least one device for generating a CO₂-rich gas flow or the plurality of devices for generating a CO₂-rich gas flow and the at least one synthesis installation (60) for producing methanol and/or secondary products of methanol from at least fractions of the CO₂-rich gas flow or the plurality of synthesis installations (60) for producing methanol and/or secondary products of methanol from at least fractions of the CO₂-rich gas flow are connected to and interconnected with the public power grid (71) as an interruptible load.

## Revendications

1. Centrale électrique (51), qui comprend une chaudière de centrale (1), équipée de brûleurs à charbon et/ou d'une turbine à gaz, comprenant un circuit eau/vapeur (54) raccordé, qui comprend au moins un groupe turbogénérateur (58) alimenté en vapeur, comprenant au moins une génératrice raccordée (70), un courant (53) de gaz d'échappement contenant du CO₂ étant produit dans la chaudière de centrale (1) équipée des brûleurs à charbon,
et qui comprend au moins un dispositif pour la production d'un courant gazeux riche en CO₂,
et qui, avec sa partie génératrice de courant, qui comprend au moins une génératrice (70), est raccordée à un réseau électrique public (71), qui met à disposition une puissance de réglage, la puissance électrique utile de la partie génératrice de courant de la centrale électrique (51) au réseau électrique (71) étant soumise à un réglage de puissance, commandé côté réseau, en particulier à un réglage primaire et/ou à un réglage secondaire et/ou à un réglage tertiaire et/ou à un réglage quaternaire,
**caractérisée en ce que**
la centrale électrique (51) comprend au moins une installation d'électrolyse (61) pour la fabrication d'hydrogène (H₂) et au moins une installation de synthèse (60) pour la fabrication de méthanol et/ou de dérivés du méthanol à partir d'au moins des fractions de CO₂ du courant gazeux riche en CO₂ et de l'hydrogène produit dans l'installation d'électrolyse (61) ; que l'au moins un dispositif de production d'un courant gazeux riche en CO₂ et l'au moins une installation d'électrolyse (61) pour la fabrication d'hydrogène (H₂) et l'au moins une installation de synthèse (60) pour la fabrication de méthanol et/ou de dérivés du méthanol à partir d'au moins des fractions CO₂ du courant gazeux riche en CO₂ et de l'hydrogène produit dans l'installation d'électrolyse (61), sont, à l'aide de lignes conductrices de courant et transporteuses de fluide, reliées et câblées par les lignes les unes aux autres de telle sorte que le courant produit côté centrale lors de l'exploitation de la centrale électrique (51) puisse en totalité ou en partie être utilisé pour l'exploitation d'un, de plusieurs ou de la totalité du groupe de dispositifs et d'installations consistant en l'au moins un dispositif de production d'un courant gazeux riche en CO₂, l'au moins une installation d'électrolyse (61) pour la fabrication d'hydrogène (H₂), et de l'au moins une installation de synthèse (60) pour la fabrication de méthanol et/ou de dérivés du méthanol.

2. Centrale électrique selon la revendication 1, **caractérisée en ce que** l'au moins une installation d'électrolyse (61) pour la fabrication d'hydrogène (H₂) ou plusieurs installations d'électrolyse (61) pour la fabrication d'hydrogène (H₂) et/ou l'au moins un dispositif pour la production d'un courant gazeux riche en CO₂ ou plusieurs dispositifs pour la production d'un courant gazeux riche en CO₂ et/ou l'au moins une installation de synthèse (60) pour la fabrication de méthanol et/ou de dérivés du méthanol à partir d'au moins des parties du courant gazeux riche en CO₂ ou plusieurs installations de synthèse (60) pour la fabrication de méthanol et/ou de dérivés du méthanol à partir d'au moins des parties du courant gazeux riche en CO₂, sont, côté centrale électrique, et pour ce qui est de leur capacité d'absorption de courant ou de puissance et de leur capacité de production d'hydrogène, ou pour ce qui concerne leur capacité d'absorption de courant ou de puissance et leur puissance de production ou de conversion, configurés et conçus avec possibilité de réglage, de telle sorte qu'il soit possible d'augmenter ou de diminuer à court terme, de préférence en quelques minutes, leur absorption de courant ou de puissance et leur production d'hydrogène et/ou respectivement leur absorption de courant ou de puissance et leur puissance de production ou de conversion, en réponse à une demande de réglage de puissance, côté réseau, envoyée à la centrale (51).

3. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs de production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂, sont, côté centrale électrique, pour ce qui est respectivement de leur capacité d'absorption de courant ou de puissance et de leur puissance de production ou de conversion, configurées et, du point de vue réglage, reliés les uns aux autres, de telle sorte qu'elles puissent être augmentées ou diminuées, à court terme, de préférence en quelques minutes, en réponse à une demande de réglage de puissance, côté réseau, envoyée à la centrale (51), pour ce qui est tant respectivement de leur consommation de courant ou de puissance que de leur puissance de production ou de conversion, de telle sorte que la centrale (51), dans le cas d'une demande côté réseau de réglage de la puissance, puisse pour ce qui est de sa puissance être adaptée à la demande de puissance modifiée, au cours d'une modification de charge présentant un gradient de modification de charge compris dans la plage de 3 %/min à 30 %/min.

4. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂, sont configurés de façon à pouvoir être individuellement sélectionnés et réglés pour ce qui est respectivement de leur absorption de courant ou de puissance et de leur puissance de production ou de conversion, et/ou sont configurés, pour ce qui est respectivement de leur absorption de courant ou de puissance et/ou de leur puissance de production ou de conversion, de telle sorte que, en particulier en réponse à une demande côté réseau de réglage de la puissance, envoyée à la centrale (51), ils puissent, en un bref laps de temps de quelques minutes, de préférence sur un laps de temps allant jusqu'à 30 minutes, être sollicités par une absorption de courant ou de puissance, qui est de 100 à 300 %, de préférence de 150 à 200 %, des valeurs correspondantes en configuration normale ou en exploitation normale de l'installation (60, 61) ou du dispositif considéré.

5. Centrale électrique selon l'une des revendications précédentes, **caractérisée en ce que** l'au moins une installation de synthèse (60) pour la fabrication de méthanol et/ou de dérivés du méthanol ou les plusieurs installations de synthèse (60) pour la fabrication de méthanol et/ou de dérivés du méthanol, sont, pour ce qui est de leur capacité, globalement configurées de telle sorte qu'il soit possible de cette manière de convertir en méthanol et/ou en un dérivé du méthanol 10 à 50 % en poids, en particulier 30 à 40 % en poids, de préférence 35 % en poids du CO₂ qui se forme à pleine charge de la centrale (51) et contenu dans le courant (53) de gaz d'échappement contenant du CO₂, et/ou sont globalement, pour ce qui est de leur capacité d'absorption de courant et de puissance et respectivement de leur puissance possible de production ou de conversion, configurées du point de vue de leur capacité de façon que le courant électrique pouvant être produit globalement par la centrale, en pleine charge et/ou en présence d'une puissance maximale de la centrale électrique (51), puisse d'une manière maximale être utilisé pour la fabrication de méthanol et/ou de dérivés du méthanol.

6. Centrale électrique selon l'une des revendications précédentes, **caractérisée en ce que** l'au moins un dispositif pour la production d'un courant gazeux riche en CO₂ ou les plusieurs dispositifs pour la production d'un courant gazeux riche en CO₂, comprennent, ou en sont constitués, au moins une installation de séparation de CO₂, en particulier une installation Post Combustion Capture (PCC) (5) et/ou un ou plusieurs brûleurs ou dispositifs de brûleurs, exploités par le procédé Oxyfuel, de la chaudière de centrale (1), avec installation associée de séparation de CO₂.

7. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène H₂ et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂, sont, pour ce qui est de leur absorption de courant ou de puissance et de leur puissance de production ou de conversion, globalement configurés de telle sorte que, lors de leur exploitation, la centrale électrique (51) puisse être exploitée sans injection de courant dans le réseau (71), dans le cas d'une exploitation avec sa charge minimale nécessaire du point de vue de la technique des installations.

8. Centrale électrique selon l'une des revendications précédentes, **caractérisée en ce que** la centrale électrique (51) est configurée comme un récepteur de courant pour le réseau électrique public raccordé (71), l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs installations pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂, pouvant, pour ce qui est de leur absorption de courant ou de puissance et de leur puissance de production ou de conversion, être globalement configurés, et câblés au réseau électrique (71), de telle sorte qu'ils puissent être exploités avec le courant excédentaire prélevé du réseau électrique (71).

9. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂, sont reliées et câblées, en tant que charge déconnectable, au réseau électrique public (71).

10. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol, sont reliés, par des lignes, à une installation de récupération de chaleur perdue qui, lors de l'exploitation de cette ou de ces installations (60, 61) et/ou de cet ou de ces dispositifs, se forme à une température comprise dans la plage de 30 à 400°C, de préférence dans la plage de 30 à 150°C, par l'intermédiaire d'au moins une ligne transportant la chaleur perdue, comportant une unité de préchauffage de l'eau d'alimentation du circuit eau/vapeur (54) et/ou une unité de préchauffage d'une installation de séparation du CO₂, en particulier une installation Post Combustion Capture (PCC) (5), et/ou une unité de préchauffage d'au moins l'une des matières de charge utilisées dans la centrale électrique (51) et/ou des produits qui y sont produits.

11. Centrale électrique selon l'une des revendications précédentes, **caractérisée en ce que** l'au moins une installation d'électrolyse (61) pour la fabrication d'hydrogène (H₂) ou les plusieurs installations d'électrolyse (61) pour la fabrication d'hydrogène (H₂) sont, pour ce qui est de leur capacité de production et/ou de conversion, configurées de telle sorte qu'il soit possible de convertir en méthanol ou en dérivés du méthanol le courant (53) de gaz d'échappement, qui se forme dans les brûleurs de la chaudière de centrale (1) avec la quantité d'hydrogène pouvant être produite de la totalité de la partie CO₂ du combustible (50) contenant du carbone lors de la combustion, et/ou, dans la ou les installations de synthèse (60), la totalité du CO₂ qui est séparée dans au moins une installation de séparation de CO₂ (5).

12. Centrale électrique selon l'une des revendications précédentes, **caractérisée en ce qu'**à chacun des dispositifs ou installations du groupe de l'au moins un dispositif pour la production d'un courant gazeux riche en CO₂, de l'au moins une installation d'électrolyse (61) pour la fabrication d'hydrogène (H₂) et de l'au moins une installation de synthèse (60) pour la fabrication de méthanol et/ou de dérivés du méthanol, est affecté au moins un réservoir de matière de charge et/ou de produit, en particulier à l'installation d'électrolyte (61) sont affectés un réservoir d'hydrogène (24) et/ou un réservoir d'oxygène (79), et au dispositif pour la production d'un courant gazeux riche en CO₂ est affecté un réservoir de CO₂(18).

13. Procédé pour l'exploitation flexible d'une centrale électrique (51) selon l'une des revendications 1 à 12, **caractérisé en ce que** l'au moins un dispositif pour la production d'un courant gazeux riche en CO₂ et l'au moins une installation d'électrolyse (61) pour la fabrication d'hydrogène (H₂) et l'au moins une installation de synthèse (60) pour la fabrication de méthanol et/ou de dérivés du méthanol à partir d'au moins les parties CO₂ du courant gazeux riche en CO₂ et de l'hydrogène produit dans l'installation d'électrolyse (61), sont reliés par des lignes, et câblés les uns aux autres, par des lignes conductrices de courant et transporteuses de fluides, de telle sorte que le courant produit côté centrale lors de l'exploitation de la centrale électrique (51) soit en totalité ou en partie utilisé au choix pour l'exploitation d'un, de plusieurs ou de la totalité du groupe de dispositifs et d'installations consistant en le dispositif pour la production d'un courant gazeux riche en CO₂, l'installation d'électrolyse (61) pour la fabrication d'hydrogène (H₂) et l'installation de synthèse (60) pour la fabrication de méthanol et/ou de dérivés du méthanol.

14. Procédé selon la revendication 13, **caractérisé en ce que** l'absorption du courant ou de puissance et la production d'hydrogène de l'au moins une installation d'électrolyse (61) pour la fabrication d'hydrogène (H₂) ou des plusieurs installations d'électrolyse (61) pour la fabrication d'hydrogène (H₂) sont, côté centrale, augmentées ou diminuées en un bref laps de temps, de préférence en quelques minutes, en réponse à une demande, côté réseau, de réglage de la puissance, envoyée à la centrale électrique (51).

15. Procédé selon la revendication 13 ou 14, **caractérisé en ce que** respectivement l'absorption de courant/de puissance et la puissance de production ou de conversion de l'au moins un dispositif pour la production d'un courant gazeux riche en CO₂ ou de plusieurs dispositifs pour la production d'un courant gazeux riche en CO₂ et/ou de l'au moins une installation de synthèse (60) pour la fabrication de méthanol et/ou de dérivés du méthanol à partir d'au moins des parties du courant gazeux riche en CO₂ ou de plusieurs installations de synthèse (60) pour la fabrication de méthanol et/ou de dérivés du méthanol à partir d'au moins des parties du courant gazeux riche en CO₂, sont augmentées ou diminuées côté centrale en un bref laps de temps, de préférence en quelques minutes, en réponse à une demande, côté réseau, de réglage de la puissance, envoyée à la centrale électrique (51).

16. Procédé selon l'une des revendications 13 à 15, **caractérisé en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂ sont individuellement commandées et réglées, pour ce qui est de leur absorption respective de courant ou de puissance et de leur puissance respective de production ou de conversion.

17. Procédé selon l'une des revendications 13 à 16, **caractérisé en ce que** l'au moins une installation d'électrolyse (61) pour la fabrication d'hydrogène (H₂) ou les plusieurs installations d'électrolyse (61) pour la fabrication d'hydrogène (H₂) et l'au moins un dispositif pour la production d'un courant gazeux riche en CO₂ ou les plusieurs dispositifs pour la production d'un courant gazeux riche en CO₂ et l'au moins une installation de synthèse (60) pour la fabrication de méthanol et/ou de dérivés du méthanol à partir d'au moins des parties du courant gazeux riche en CO₂ ou les plusieurs installations de synthèse (60) pour la fabrication de méthanol et/ou de dérivés du méthanol à partir d'au moins des parties du courant gazeux riche en CO₂ sont exploités sous forme d'une charge déconnectable, reliée et câblée au réseau électrique public (71).
